# EUROPEAN PATENT SPECIFICATION

(11) **EP 4 326 200 B1**
(45) Date of publication and mention of the grant of the patent: **21.10.2026**
(21) Application number: 22729284.4
(22) Date of filing: 16.04.2022
(51) Int. Cl.: A61F 2/30, A61F 2/28, A61F 2/38

(54) **MOLD FOR THE FORMING OF A SPACER DEVICE FOR THE REPLACEMENT OF AN ARTICULAR PROSTHESIS**
FORM ZUR BILDUNG EINER ABSTANDHALTERVORRICHTUNG ZUM ERSATZ EINER GELENKPROTHESE
MOULE POUR LA FORMATION D'UN DISPOSITIF D'ESPACEMENT POUR LE REMPLACEMENT D'UNE PROTHESE ARTICULAIRE

(30) Priority: 19.04.2021 IT 202100009902
(43) Date of publication of application: 28.02.2024
(60) Divisional application: 25205419.2 / 4 656 164
(73) Proprietor: TECRES S.P.A., 37066 Sommacampagna (VR) (IT)
(72) Inventor: CAIAZZA, Emanuele, 00156 Roma (IT)
(74) Representative: Feltrinelli, Secondo Andrea
(86) International application number: PCT/IB2022/053584
(87) International publication number: WO 2022/224115

(56) References cited:
- WO-A1-2009/076168
- WO-A1-2013/086177
- WO-A1-2016/071939
- US-A1- 2016 332 328
- US-B2- 8 920 152
- US-B2- 9 433 506

## Description

### TECHNICAL FIELD OF THE INVENTION

The present invention relates to a mold for forming a spacer device for the replacement of a joint prosthesis.

### STATE OF THE ART

The material with which the spacer devices for the replacement of a joint prosthesis are usually made is a material known, for example, as bone cement, which initially appears in a fluid state and hardens after a certain period of time. Once hardened, such material constitutes the structure of the spacer device.

The forming of the spacer device thus takes place through special molds which can be used directly by the surgeon in the operating theatre or also at external manufacturing sites which are then responsible for providing the surgeon, who has to perform the implant, with a preformed and substantially ready-to-use spacer device.

In the first case, doctors often use flexible molds which they fill with bone cement in a fluid state and from which, following solidification of the bone cement, they extract the spacer device formed by elastic or non-elastic deformation of the material making up the mold.

Precisely because of the deformability of the material that constitutes the mold, however, the resulting spacer devices may not have the desired configuration or may have defects that, before implantation in the surgical site, must be eliminated by further processing, for example by smoothing, and the like.

There are other types of mold, made of rigid material, which make possible the formation of spacer devices of the desired configuration. However, it is often difficult to extract the spacers from these molds after the bone cement has hardened. In addition, spacer devices made with such molds also require subsequent finishing steps before their actual use. Furthermore, it is important that the molds are of a disposable type in order to ensure optimal sterility and to guarantee that the cavity therein, which determines the final configuration of the resulting spacer device, has not been compromised in any way or has not undergone any modifications from a previous use such that the spacer device cannot be obtained in the desired shape. This is not always possible with traditional molds. Patent application US 2016/332328 describes a mold for forming a hip spacer comprising a base element, wherein there are provided a hollow space as a negative image of a femoral stem and a recess connected to the hollow space, a hemispherical insert for molding a side of a femoral head of the spacer, to be housed in the recess, and an adapter insert shaped as a hollow body open on both sides, to be inserted into the recess between the hollow space and the hemispherical insert. The mold also includes a punch component which also comprises a hollow space as a negative image of the remaining part of the femoral stem.

There is therefore a need for a new type of mold for forming spacer devices for the replacement of a joint prosthesis that overcomes the drawbacks of the prior art.

### PURPOSES OF THE INVENTION

The main purpose of the present invention is thus to improve the state of the art in the field of molds for forming spacer devices for the replacement of a joint prosthesis. Another purpose of the present invention is to provide a mold for forming a spacer device that is quick and easy to use.

A further purpose of the present invention is to provide a mold for forming a spacer device that allows molding of the device in question by means of a substantially single operating step, without the need to perform further processing or finishing steps on the device obtained by means of the same.

Yet another purpose of the present invention is to provide a mold for forming a spacer device that can be manufactured at a competitive price.

A still further purpose of the present invention is to provide a mold for forming a spacer device that allows the doctor to choose the most suitable material for forming the mold itself, for example, an antibiotic mixture calibrated to the real needs of the patient and prepared, for example, on the instructions of a pharmacologist, based on the latter's identification of the specific patient's pathogenic flora.

A further purpose of the present invention is to provide a mold for forming a spacer device that is provided with an alternative configuration with respect to the state of the art. According to an aspect of the present invention, a mold is provided for forming a spacer device for the replacement of a joint prosthesis according to claim 1. According to an aspect of the present invention, a method is provided for forming a spacer device for the replacement of a joint prosthesis according to claim 22.

The dependent claims refer to preferred and advantageous embodiments of the invention.

### BRIEF DESCRIPTION OF THE DRAWINGS

Further features and advantages of the present invention will become more evident from the detailed disclosure of some preferred exemplary embodiments of a mold for forming a spacer device, illustrated by way of example only, in the appended drawings, wherein:
Figure 1 shows a perspective view of a mold for forming the tibial knee spacer device, in a partially open configuration;
Figure 2 shows a plan view of the mold of Figure 1 in a fully open configuration;
Figure 3 shows a side view of the mold of Figure 2;
Figure 4 shows a sectional view taken along the A-A section plane of the mold of Figure 2;
Figure 5 shows an enlarged detail of a component of the mold of Figure 4;
Figure 6 shows an enlarged detail of a component of the mold of Figure 3;
Figure 7 shows an exploded view of the mold of Figure 1;
Figures 8A and 8B show perspective views, from above and below, respectively, of a tibial spacer device obtainable with the mold of the previous figures;
Figure 9 shows a perspective view of a mold for forming the femoral knee spacer device, in a partially open configuration;
Figure 10 shows a plan view of the mold of Figure 9 in a fully open configuration;
Figure 11 shows a side view of the mold of Figure 10;
Figure 12 shows a sectional view taken along the A-A section plane of the mold of Figure 10;
Figure 13 shows an exploded view of the mold of Figure 9;
Figure 14 shows an enlarged detail of a component of the mold of Figure 11;
Figure 15 shows an enlarged detail of a component of the mold of Figure 11;
Figures 16A and 16B show perspective views, from below and above respectively, of a femoral spacer device obtainable with the mold of the preceding Figures 9 to 15;
Figure 17 shows a perspective view of a mold for forming a hip spacer device, in a partially open configuration;
Figure 18 shows a plan view of the mold of Figure 17 in a fully open configuration;
Figure 19 shows a side view of the mold of Figure 18;
Figure 20 shows an exploded view of the mold of Figure 17;
Figure 21 shows an enlarged detail of a component of the mold of Figure 19;
Figures 22A and 22B show side views of two examples of hip spacer devices obtainable with the mold of the preceding Figures 17 to 21;
Figure 23 shows a perspective view of a mold for forming a shoulder spacer device, in a partially open configuration;
Figure 24 shows a plan view of the mold of Figure 23 in a fully open configuration;
Figure 25 shows a side view of the mold of Figure 24;
Figure 26 shows an enlarged detail of a component of the mold of Figure 25;
Figure 27 shows an exploded view of the mold of Figure 23;
Figures 28A and 28B show side views of two examples of shoulder spacer devices obtainable with the mold of the preceding Figures 23 to 27;
Figure 29 shows a sectional perspective view of the mold for forming the tibial knee spacer device of Figure 1, in a closed configuration, taken along a section plane coinciding with the A-A section plane of Figure 2;
Figure 30 shows a side view of the mold of Figure 29;
Figures 31 and 32 show enlarged details of Figure 29 and Figure 30, respectively;
Figures 33 and 34 show, respectively, a plan view and a sectioned enlarged detail taken along the A-A section plane of Figure 33 of a further version of a mold for forming the tibial knee spacer device;
Figures 35 and 36 show, respectively, a plan view and a sectioned enlarged detail taken along the A-A section plane of Figure 35 of a further version of a mold for forming the femoral knee spacer device;
Figures 37 and 38 show, respectively, a plan view and an sectioned enlarged detail view taken along the A-A section plane of Figure 37 of a further version of a mold for forming a hip or shoulder spacer device;
Figure 39A shows a perspective view of another version of a mold for forming the femoral knee spacer device in a closed configuration;
Figure 39B shows an enlarged detail from Figure 39A;
Figure 40 shows a plan view of the mold of Figure 39A in an open configuration;
Figure 41A shows a sectional view taken along the A-A section plane of Figure 40;
Figure 41B shows an enlarged detail from Figure 41A;
Figures 42A and 42B show plan views of the mold of Figure 39A;
Figure 43A shows a front view of the mold of Figure 39A;
Figure 43B shows a sectional view taken along the C-C section plane of Figure 43A;
Figure 44 shows an exploded view of the mold of Figure 39A;
Figure 45A shows a plan view of a component of the mold of Figure 39A;
Figure 45B shows a sectional view taken along the A-A section plane of Figure 45A;
Figure 45C shows an enlarged detail of Figure 49B;
Figure 46A shows a side view of the component of Figure 45A;
Figure 46B shows a sectional view taken along the B-B section plane of Figure 46A;
Figures 47A and 47B show, respectively, a front and perspective view of an element of the mold of Figure 39A.

In the accompanying drawings, identical parts or components are identified by the same numerals.

### EMBODIMENTS OF THE INVENTION

The present invention relates to a mold for forming a spacer device for replacing a joint prosthesis.

As it is usual, the spacer device is a temporary, disposable device which is implanted in the human body, for example at a joint in the human body, to replace a permanent joint prosthesis. This occurs basically because the implant site of the permanent joint prosthesis is infected and it becomes necessary, on the one hand, to treat the infection after removing the infected prosthesis and, on the other, to protect the joint space until a new prosthesis is implanted in the surgical site from which the infection has been removed. These functions are performed by the spacer device, which is made of a suitable biocompatible material, such as a bone cement based on an acrylic resin, for example polymethyl methacrylate, possibly with the addition of at least one medical substance with an antimicrobial effect (for example, at least one antibiotic to treat the infection at the implant site). The most commonly used antibiotics are one of gentamicin, vancomycin, and so forth, or combinations thereof.

This material, in at least one version of the invention, comprises bone cement, based, for example, on polymethyl methacrylate (PMMA).

The antibiotic or blend of antibiotics is mixed with bone cement by the doctor on the basis of, for example, the indications of the pharmacologist who has evaluated the specific bacterial or pathogenic flora of the patient. The resulting material or mass is used to form the most effective spacer device for eradicating the infection in the specific patient.

In the following disclosure, the terms "upper" and "lower", and the like, refer to a specific configuration (e.g. opening or closing) of the mold according to the present invention, and do not necessarily remain such when the mold changes to a configuration other than that for which said terms have been indicated.

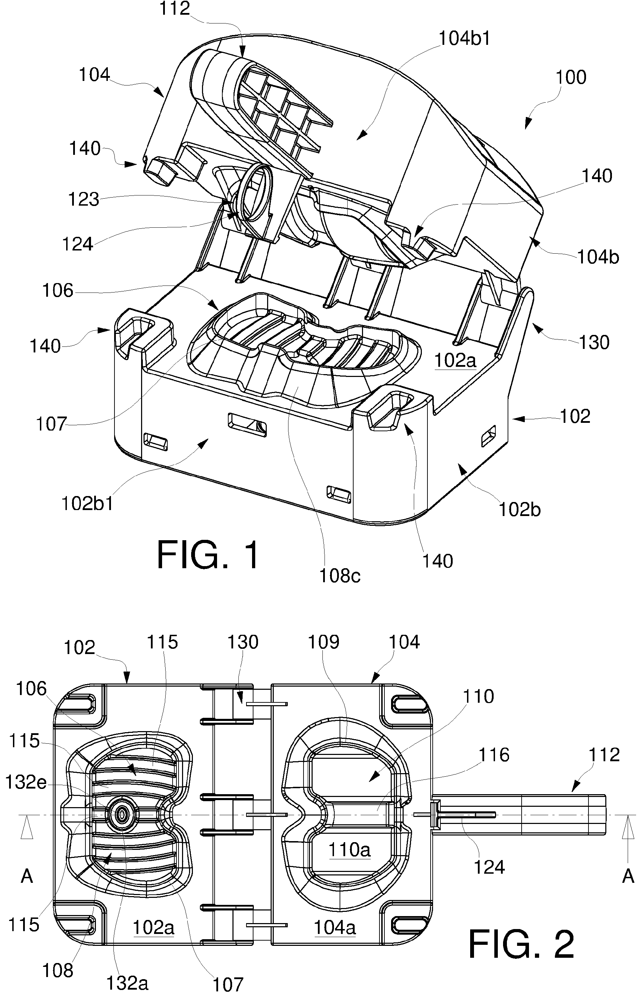

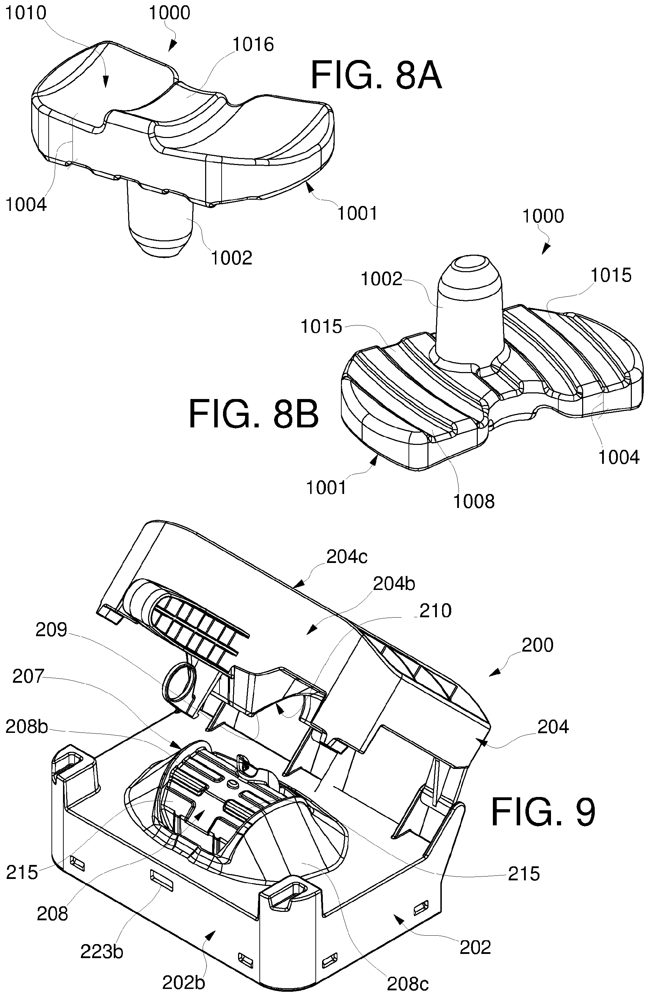

Figures 1 to 7 show a mold 100 for forming a tibial knee spacer device (the latter shown in Figures 8A and 8B), adapted for implantation in use at the tibial bone of the knee joint;

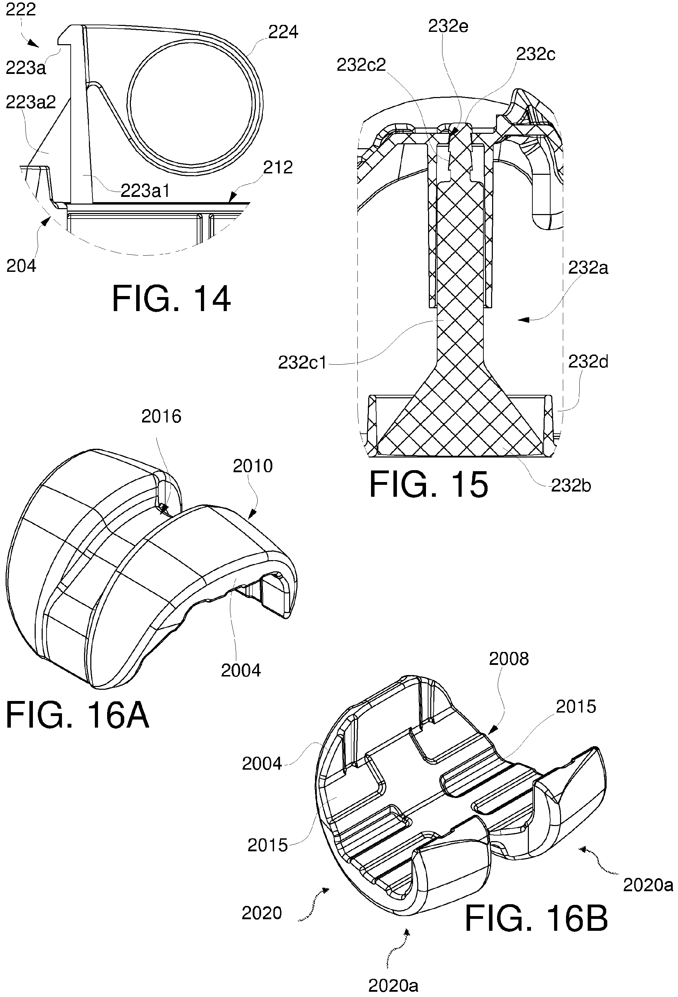

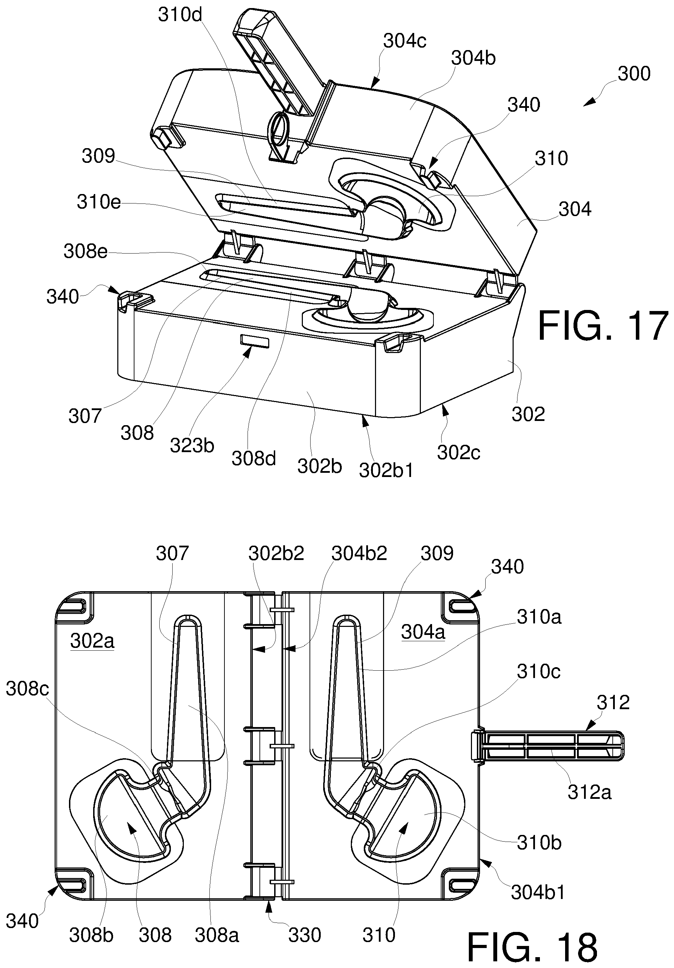

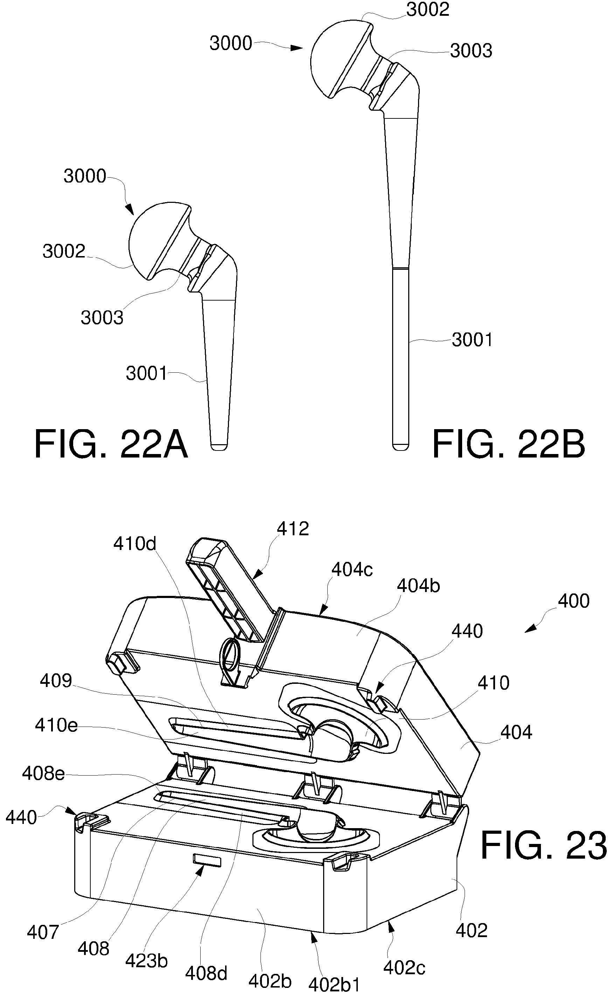

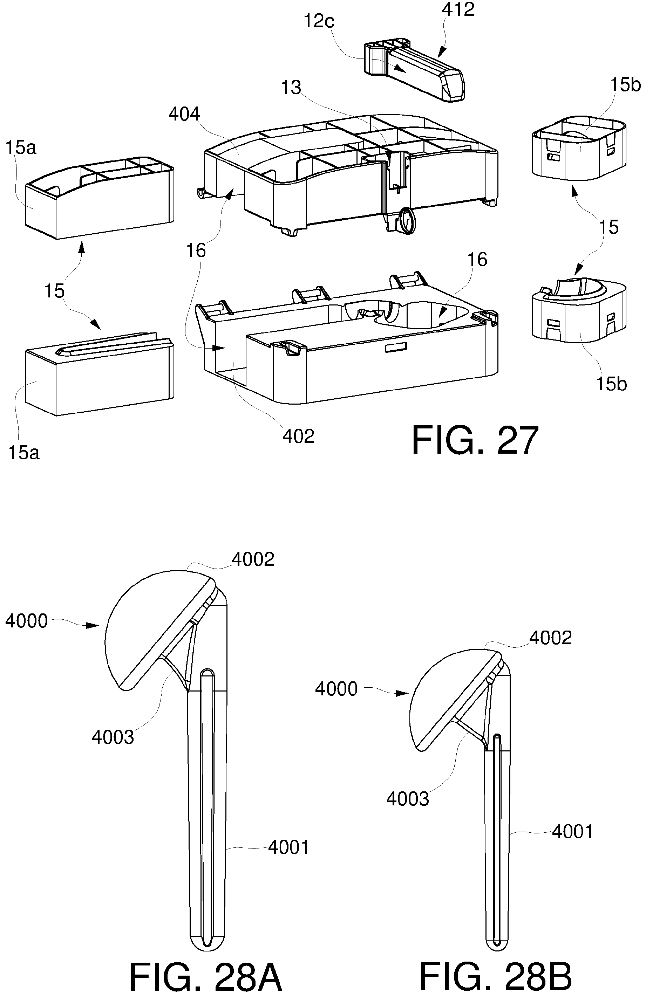

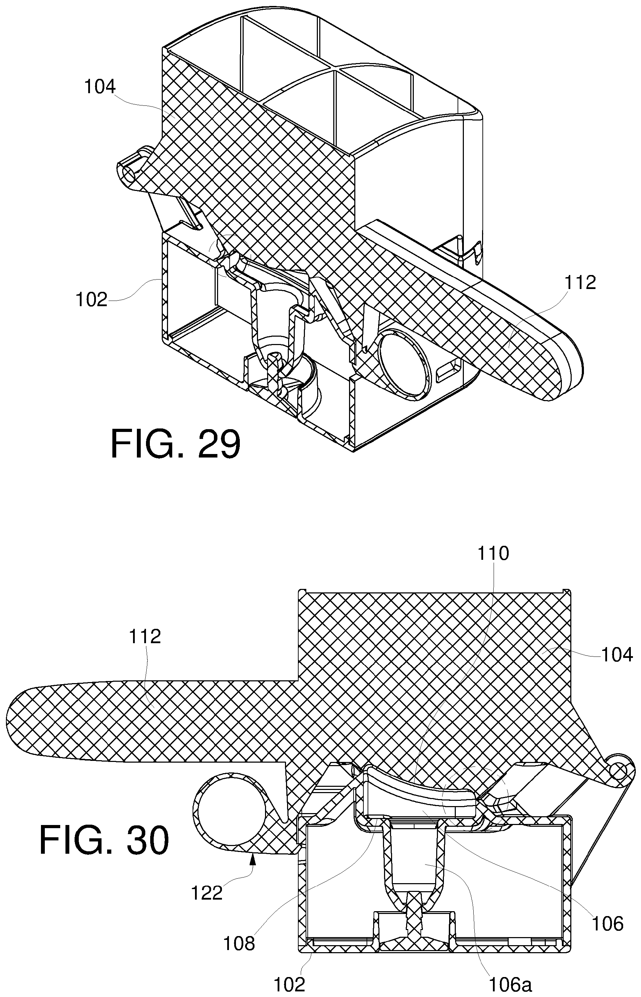

Figures 9 to 15 show a mold 200 for forming the femoral knee spacer device (the latter shown in Figures 16A and 16B), adapted to be implanted in use at the femoral bone of the knee joint; Figures 29A to 47B show another version of the mold 200 for forming the femoral knee spacer device (the latter shown in Figures 16A and 16B); Figures 17 to 21 show a mold 300 for forming a hip spacer device (the latter shown in Figures 22A and 22B), adapted for implantation in use at the femoral bone of the hip joint; and Figures 23 to 27 show a mold 400 for forming a shoulder spacer device (the latter shown in Figures 28A and 28B), adapted in use for implantation at the shoulder joint.

At any rate, and as already mentioned, the present invention generally refers to a mold for forming a spacer device and, therefore, unless otherwise explicitly stated, what is disclosed for one version of the invention can also be applied to another version of the invention.

In general, the mold according to the present invention has a shell structure composed of at least two half-shells, that is, at least one first half-shell and one second half-shell. The shell structure is a rigid structure, meaning it does not deform when subjected to manual force and/or any reaction forces that develop during the forming of the spacer device in question.

In at least one version of the invention, the material that constitutes the mold, although rigid, is free of additives that could be extracted and/or absorbed by the bone material or cement that will constitute the spacer device during the forming. The extraction and/or absorption of the additives present in the mold is a very serious problem that is present, for example, in molds made of elastomers, such as silicone, and so forth. As is evident, if the material of the molded spacer device has in its mass any additives extracted and/or absorbed by the material constituting the mold, this undermines the safety of the spacers molded through such molds from a toxicological point of view. Since 2018, many molds of this type have been withdrawn from the global market.

In one version of the invention, the mold is made of polypropylene.

As will be apparent from the following disclosure, in at least one version of the invention the two half-shells differ from one another in at least one feature. Hence, unless otherwise explicitly stated, the first half-shell and the second half-shell do not mirror each other. Regarding the forming step, the first half-shell is also called the lower body while the second half-shell is also called the upper body, one with respect to the other, and considering the support plane on which the mold can rest in the closed configuration during the forming step of the respective spacer device. The second half-shell can therefore act as a cover for the first half-shell.

During the forming step, the mold is in fact in a closed operating configuration, in which the first half-shell is placed on top of second half-shell.

During the loading step of the material the spacer device is made of, however, the mold is in an open, e.g. book-like, operating configuration. In this case, the first and the second half-shells can both rest on a support plane and/or be placed side by side.

With respect to the version illustrated in Figures 1 to 8, the mold 100 comprises a first half-shell or lower body 102 and a second half-shell or upper body 104.

The first half-shell 102 and the second half-shell 104 are adapted to be coupled in use so as to define therebetween a cavity 106 corresponding to the external configuration of the spacer device that they are designed to form.

Therefore, the cavity 106 corresponds to the external configuration of the whole spacer device that is designed to form.

In particular, the first half-shell 102 and the second half-shell 104 respectively comprise a first forming surface 108 and a second forming surface 110. When the mold is closed, the first forming surface 108 is placed on top of the second forming surface 110 so as to form the cavity 106.

The cavity 106 and/or the first forming surface 108 and/or the second forming surface 110 are substantially C-shaped in the plan view, corresponding to that which the resulting tibial spacer device will have.

In particular, the free ends of the C are located, as far as the first half-shell 102 is concerned, at a rear area thereof. However, a different position is possible, without departing from the purpose of the present invention.

The first forming surface 108 is delimited by a first perimeter edge 107 while the second forming surface 110 is delimited by a second perimeter edge 109.

The first forming surface 108 and/or the second forming surface 110 make up the area of application of the material that will constitute the spacer device to be formed with the mold according to the present invention and are respectively intended to shape at least a first portion and at least a second portion of the spacer device. When the mold is in open configuration, both the first and second forming surfaces are accessible from the outside. **In** at least one version of the invention, the first and second forming surfaces 108, 110 are adapted in use to form two cross-sectional portions (i.e. substantially parallel to a transverse plane of the human body) or two longitudinal portions (i.e. substantially parallel to the longitudinal axis) of the resulting spacer device.

**In** at least one version of the present invention, the first forming surface 108 determines the forming of (and/or is adapted to form in use) a first in use lower portion of the resulting spacer device (in the sense that, at the time of implantation, it will be positioned lower than the human body considered in an upright position).

Similarly, the second forming surface 110 determines the formation of (and/or is adapted to form in use) a second in use upper portion of the resulting spacer device (in the sense that, at the time of implantation, it will be positioned higher than the human body considered in an upright position).

Additionally or alternatively, the first portion can comprise surfaces of the spacer device adapted to come into contact with the patient's bone when implanted, while the second portion can comprise articulating surfaces (for a further spacer component or for a patient's bone) of the resulting spacer device.

Accordingly, the first forming surface 108 is adapted in use to form substantially only patient bone contact surfaces in use of the resulting spacer device while the second forming surface 110 is adapted in use to form substantially only articulating surfaces in use of the resulting spacer device (or vice-versa).

It follows that, in the closed operating step of the mold, considering the spacer device to be formed, the first portion and the second portion are joined together on a plane parallel to a transverse plane of the human body.

Therefore, generally speaking, the first perimeter edge 107 and the second perimeter edge 109 lie - in the closing position of the mold and/or when they are in contact with each other - on a plane parallel to a transverse plane of the human body or on a plane parallel to a transverse plane of the spacer device to be formed.

The first perimeter edge 107 and the second perimeter edge 109 are adapted in use to determine a transverse peripheral profile of the resulting spacer device.

In use, the first perimeter edge 107 is adapted to be brought into abutment against the second perimeter edge 109. During the forming step, therefore, the first perimeter edge 107 is adapted to come into contact with the second perimeter edge 109.

The first forming surface 108 and the second forming surface 110 determine the cavity 106 for forming of the spacer device when the mold is closed. Indeed, the first half-shell 102 and the second half-shell 104 engage in a removable way with each other at the respective first perimeter edge 107 and second perimeter edge 109, thereby delimiting the cavity 106 between them.

The first perimeter edge 107 and the second edge 109 extend along the entire perimeter of the first forming surface 108 and the second forming surface 110 respectively (and thus along the entire transverse profile, for example, of the resulting spacer device). In this way, when the first perimeter edge 107 and the second perimeter edge 109 are in contact with each other because the mold 100 is closed, they leave no contact-free zone between them from which material for forming the spacer device could possibly escape, for example by forming machining burrs, without at least one of the first perimeter edge 107 and the second perimeter edge 109 being able to act on said material, for example by cutting it.

Thus, the first perimeter edge 107 is continuous and unique, as well as the second perimeter edge 109 is continuous and unique, for the entire first forming surface 108 and for the second forming surface 110.

In addition, with the exception of the depth gauges 140, which will be discussed below, the first half-shell 102 and the second half-shell 104, in at least one embodiment, come into contact with each other only at the first perimeter edge 107 and the second perimeter edge 109. The latter, therefore, constitute the maximum contact zone between the first half-shell 102 and the second half-shell 104, when the mold 100 is closed. They also constitute the minimum necessary surface of abutment for the correct forming of the spacer device which has to be molded.

In at least one version of the invention, the first perimeter edge 107 and the second perimeter edge 109 have a circular and/or ring-shaped and closed pattern.

In fact, the first forming surface 108 and the second forming surface 110 are respectively two surfaces that, while having a conformation corresponding to that of the spacer device to be formed, are surfaces enclosed by the first perimeter edge 107 and the second perimeter edge 109 that are continuous all along their perimeter.

With particular reference to the Figures indicated, the first forming surface 108 is adapted to form a portion or lower face 1008 of the tibial knee spacer device 1000, the latter being illustrated in Figures 8A and 8B, while the second forming surface 110 is adapted to form a portion or upper face 1010 of the spacer device 1000.

The upper face 1010 is adapted in use to be articulated with a femoral knee spacer device (such as that illustrated in Figures 16A and 16B or a generic femoral knee spacer device) or with the femoral end of a knee joint of a patient, while the lower face 1008 is adapted in use to be implanted at the end of the patient's tibial bone at the knee joint.

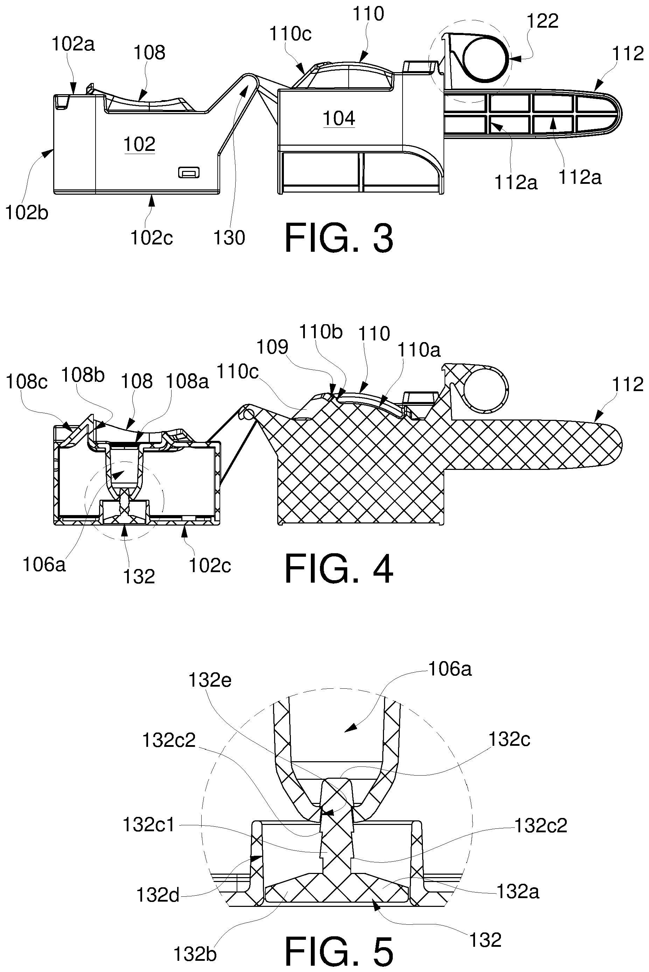

The spacer device 1000 may also comprise a stem 1002, possibly formed by means of a special additional cavity 106a (visible, for example, in Figure 4 and partially in Figure 5) placed at the first forming surface 108.

The cavity 106 therefore substantially determines the formation of the tibial plate 1001 that has a substantially rectangular or oval or C-shaped conformation in the plan view and a thickness 1004 given by the distance between the lower face 1008 and the upper face 1010 of the spacer device 1000. The cavity 106a substantially determines the shaping of the stem 1002. However, the cavity 106 and the additional cavity 106a are contiguous to each other and/or in fluid connection, so that the material that will form the spacer device 1000 can be inserted and/or flow (during the forming stage) into both said cavities 106, 106a and, in one step, form both the tibial plate 1001 and the stem 1002 of the spacer device 1000.

In an alternative version of the invention, the first half-shell 102 and the second half-shell 104 are substantially the same and mirror each other. It follows that the first forming surface 108 and the second forming surface 110 are substantially the same and mirror each other.

In this case, the first molded portion and the second molded portion of the resulting spacer device are identical and mirror each other, joined at a median plane of symmetry (hence parallel to a sagittal plane of the human body). In use, the first peripheral edge 107 and the second peripheral edge 109 of the mold 100 lie along the median plane of symmetry of the resulting spacer device.

In this version, in the resulting spacer device both the first and the second forming surfaces are each adapted to form contact surfaces with the patient's bone and joint surfaces.

A handle 112, possibly removable, is located at the second half-shell 104 of the mold 100. The surgeon or the personnel in charge of forming the spacer device 1000 by means of the mold 100 will in fact use this handle 112 to bring the first half-shell 102 into abutment against the second half-shell 104 (or vice-versa) and to apply adequate force to compress the material that will constitute the spacer device itself. Indeed, the mold 100 is not an injection mold and the loading of the material into the mold 100 will be illustrated in greater detail in the continuation of the present disclosure.

For this purpose, the step of filling the mold with the material that will constitute the spacer device, or with the bone cement, does not require auxiliary accessories such as injection or pressurisation systems (specific syringes).

On the other hand, these accessories are necessary for the molds currently available on the market and often involve an additional cost and a definite complication in use, and increase the defectiveness of the molded device due to the presence of bubbles associated with the use of syringes. Therefore, filling these injection molds necessarily requires a very fluid cement that is injected, flows smoothly but, while filling the mold, encapsulates air that creates bubbles of various sizes. Following hardening of the cement, these bubbles leave many cavities, hence negative defects, on the surface of the molded device, making its shape incomplete.

In contrast, with the mold of the present invention, filling is done manually, using, for example, a cement which has reached a high ideal viscosity established by a standard test called "doughing time". Doughing time establishes the right viscosity so that the surgeon can take the cement from the mixing vessel and manipulate it for application without damaging it (the test is defined by ISO 5833/1 Second edition 2002-05-01 Implants for surgery- Acrylic resin cements- Implant chirurgicaux - Ciment à base de rèsine acrylique, page 7). This condition, easily repeatable in all operating theatres in which the bone cement and/or the mold according to the present invention is used, makes possible the safe use of the cement while obtaining a resulting defect-free molded device.

This is an important simplification of use because it takes advantage of traditional bone cement preparation while achieving the desired results.

The handle 112 has a substantially elongated and possibly ergonomic conformation, so as to be easily gripped by an operator's hand. The shape of said handle 112, therefore, has a substantially rectangular longitudinal section, with bevelled edges (as seen, for example, in Figure 4), while the cross section thereof can be substantially rectangular, square, circular, triangular, and so forth.

By "longitudinal section" is meant that section taken along a plane that is perpendicular to the mold along the longest dimension of the handle, while by "cross section" is meant that section taken along a plane that is perpendicular to the mold along the shortest dimension of the handle.

The handle 112 may internally comprise reinforcing elements 112a, for example ribs (as shown in Figure 3), in order to ensure better resistance to the forces applied to close the two half-shells 102, 104 of the mold 100 and the consequent forming of the spacer device 1000.

The first half-shell 102 and/or the second half-shell 104 have a substantially box-like conformation, for example a substantially polyhedral and/or parallelepipedal, possibly rectangular or cylindrical conformation. The first half-shell 102 and/or the second half-shell 104 may have a casing and/or block configuration, open from at least one side, or closed and/or may be full or internally hollow.

The faces of the polyhedron and/or the parallelepiped can, for example, be substantially triangular, square or rectangular, possibly with bevelled edges, and so forth. Two faces are of larger size and lie on two planes parallel to each other and parallel to the support plane on which the mold 100 is adapted to be placed, while the other at least three faces, four in the attached Figures, constitute a side wall which develops along the perimeter of at least one of the faces of larger size, in a substantially perpendicular manner to the latter. The side wall therefore consists of smaller dimensions faces.

In the case of a cylindrical conformation, on the other hand, there are two substantially larger, circular or oval faces and a side wall, possibly tubular, extending along the perimeter or circumference of at least one of the larger faces.

Thus, the first half-shell 102 comprises at least one upper face 102a, adapted to house the first forming surface 108 and a side wall 102b. There may also be a lower face 102c; alternatively, the first half-shell 102 may be open at the bottom (for example, the lower face 102c may have an opening). The upper face 102a and, possibly, the lower face 102c constitute the larger faces of the first half-shell 102.

The upper face 102a has the first forming surface 108 in a substantially central position. The first forming surface 108 consists of a first base 108a, in recess with respect to the upper face 102a and/or the first perimeter edge 107, and a first side surface 108b. The side surface 108b extends along the perimeter of the base 108a, from the latter to the first perimeter edge 107. The side surface 108b is perpendicular to or inclined towards the outside with respect to the base 108a. The first perimeter edge 107 is therefore raised with respect to the base 108a and/or the upper face 102a.

In general, at least one of the first perimeter edge 107 and the second perimeter edge 109 is raised with respect to the first half-shell or lower body 102 and the second half-shell or upper body 104, respectively.

Naturally, together with the base 108a, the side surface 108b also contributes to the shape of the tibial spacer device resulting from forming in said mold 100. Thus, the base 108a and the side surface 108b will be conformed correspondingly, albeit negatively, to at least the lower face 1008 and to at least part of the thickness 1004 of the tibial plate 1001. Furthermore, at the substantially centrally positioned base 108a, there is an opening to the additional cavity 106a for the stem 1002 of the spacer device in question.

In particular, the base 108a may have a series of first ribs 115 adapted to determine the formation of respective first longitudinal grooves 1015 of the lower face 1008 of the tibial plate 1001.

The upper face 102a, therefore, is connected to the first perimeter edge 107 by a first raised wall 108c. The raised wall 108c is substantially inclined or perpendicular with respect to the upper face 102a and, in at least one version of the invention, moves away from the first perimeter edge 107 and/or from the base 108a and/or from the first forming surface 108.

In at least one version of the invention, the remaining part of the upper face 102a is flat and substantially coplanar.

In a substantially similar way, the second half-shell or upper body 104 comprises at least one lower face 104a, adapted to house in a substantially central position the second forming surface 110 and a side wall 104b. An upper face 104c may also be present; alternatively, the second half-shell 104 may be open at the top.

The second forming surface 110 consists of a (second) base 110a, which rises with respect to the lower face 104a. In one version of the invention and/or in at least some areas thereof, the base 110a rises from the lower face 104a to a greater extent than the second perimeter edge 109. The latter can therefore be positioned between the most protruding point of the base 110a and the lower face 104a.

The second half-shell 104 further comprises a (second) side surface 110b. The side surface 110b extends along the perimeter of the base 110a, from the latter to the second perimeter edge 109. The lateral surface 110b is perpendicular to or inclined towards the outside with respect to the base 110a. The second perimeter edge 109 is thus raised with respect to the lower face 104a and/or in general with respect to the second half-shell 104. Naturally, together with the base 110a, the side surface 110b also contributes to the shape of the tibial spacer device resulting from forming in said mold 100. Thus, the base 110a and the side surface 110b will be shaped in a manner corresponding, albeit negatively, to at least the upper face 1010 and to at least part of the thickness 1004 of the tibial plate 1001.

In particular, the base 110a can have a substantially smooth surface, although, in at least one version of the invention, comprising a longitudinal central recess 116 corresponding to a substantially rectangular base protrusion 1016 present at the upper face 1010 of the tibial plate 1001.

The lower face 104a is thus connected to the second perimeter edge 109 by a (second) raised wall 110c. The raised wall 110c is substantially inclined or perpendicular to the lower face 104a and, in at least one version of the invention, moves away from the second perimeter edge 109 or the second forming surface 110.

In at least one version of the invention, the remaining part of the lower face 104a is flat and substantially coplanar.

At the side wall 102b of the first half-shell 102 and the side wall 104b of the second half-shell 104, it is possible to identify a (first) front in use area 102b1 and a (second) front in use area 104b1 and a (first) rear in use area 102b2 and a (second) rear in use area 104b2. In a version of the invention, the rear in use area 102b2 and 104b2 is opposite the front in use area 102b1 and 104b1.

The handle 112 can be positioned at the front in use area 104b1 of the second half-shell 104.

In addition, a removable constraining structure 122 can be positioned at this area, adapted to be constrained in a removable way to the first half-shell 102.

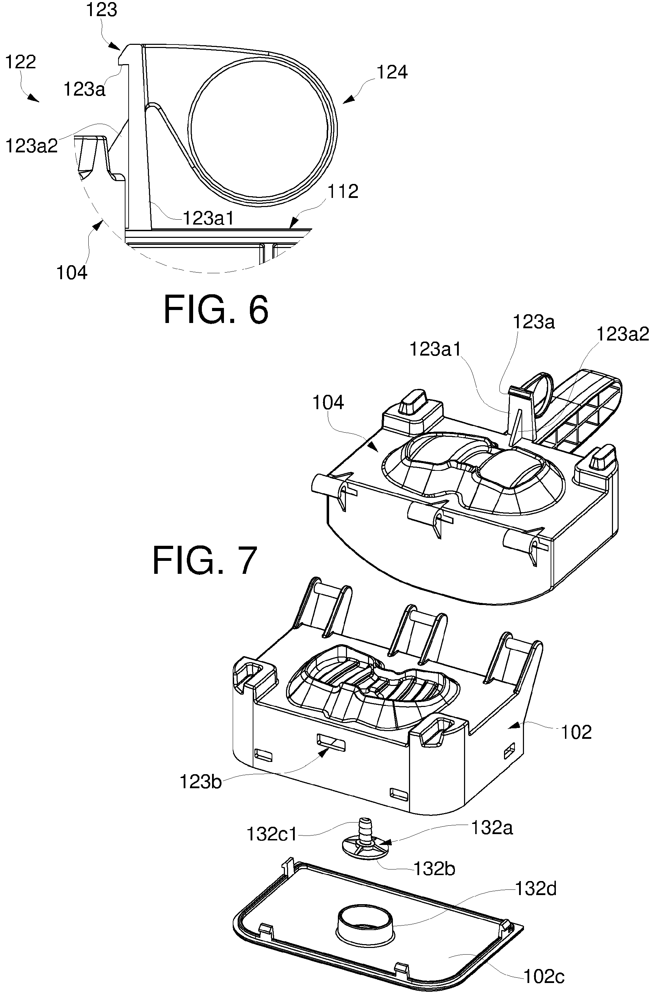

In particular, as illustrated for example in the enlarged detail of Figure 6 (taken from Figure 3), the removable constraining structure 122 comprises coupling means 123, for example snap-on, and possibly a trigger element 124.

In at least one version of the invention, the removable constraining structure 122 also comprises the handle 112.

The removable coupling means 123 include, for example, a tooth or protrusion element 123a, adapted to engage in a removable way in a suitable engagement seat 123b. The engagement seat 123b is placed, for example, at the front in use area 102b1 of the side wall 102b of the first half-shell 102.

Naturally, it is also possible for the coupling means 123 to be placed in a different position than that indicated above. For example, the tooth or protrusion element 123a could be positioned at the first half-shell 102 and the engagement seat 123b could be positioned at the second half-shell 104.

The coupling means 123 may comprise a special bracket 123a1 adapted to carry the tooth or protrusion element 123a. Said bracket 123a1 has an elongated conformation that departs from the second half-shell 104 for a length such as to be able to bring the tooth or protrusion element 123a to the engagement seat 123b, when the second half-shell 104 is placed on top of the first half-shell 102 to close the mold 100 and then start the forming step of the spacer device 1000.

This bracket 123a1 has, for example, a substantially rectangular shape in the plan view, and a length greater than its width, in turn greater than its thickness. Thus, the bracket 123a1 may be long and thin.

As previously mentioned, a first end or zone of the bracket 123a1 is stably constrained to the second half-shell 104 and/or the handle 112, while a second end or zone thereof, opposite the first end or zone, for example further away than the second half-shell 104, carries the tooth or protrusion element 123a.

The tooth or protrusion element 123a is present in the first face of the bracket 123a1 which, when the mold 100 is closed, faces in use the first half-shell 102.

In a position opposite the tooth or protrusion element 123a, and therefore for example on the second face of the bracket 123a1, opposite the first face, second face which, in use, when the mold is closed, is facing away from the first half-shell 102, the trigger element 124 may be positioned. Otherwise, the trigger element 124 may be carried directly by the handle 112 or other suitable structure.

When the operator in charge of forming the spacer element closes the mold, bringing the first half-shell 102 and the second half-shell 104 into abutment against each other so as to form the cavity 106 between the first forming surface 108 and the first perimeter edge 107 and the second forming surface 110 and the second perimeter edge 109, he/she does so using the handle 112. By applying a for example manual force on the handle 112, he/she can hook on the tooth or protrusion element 123a, for example inside the engagement seat 123b, due in part to the bracket 123a1.

Once the forming step has been completed, and therefore after having waited a predetermined time for the hardening of the material that constitutes the spacer device, the operator acts on the trigger element 124 to determine the release and/or uncoupling of the tooth or protrusion element 123a from the engagement seat 123b. Since the trigger element 124 is in the vicinity of the handle 112, the operator can, in substantially one-step, grip the handle 112, release the coupling means 123 using the trigger 124, move the second half-shell 104 away from the first half-shell 102 and, by rotating with these two with respect to each other, again using the handle 112, cause the mold 100 to open.

The bracket 123a1 may further comprise a stiffening rib 123a2, for example sail-shaped or triangular, located at the first face of the bracket 123a1. This stiffening rib 123a2 helps to support the force applied by the operator to close the mold 100, as well as the force needed during the forming step to keep the mold closed.

The engagement seat 123b, for example having a rectangular or circular opening placed at the wall of the half-shell in question, is shaped in a substantially corresponding and/or complementary manner to the tooth or protrusion element 123a, so that when the mold 100 is closed, for example by acting on the handle 112, possibly manually, the tooth or protrusion element 123a is inserted, possibly snapped-on, inside the engagement seat 123b.

Connection between the engagement seat 123b and the tooth or protrusion element 123a is also obtained on account of the deformability of the material that makes possible the movement toward the engagement seat 123b of the tooth or protrusion element 123a with the insertion and/or coupling and/or snapping of the same into the engagement seat 123b. **In** this way, a temporary constraint is created between the first half-shell 102 and the second half-shell 104 that makes possible a perfect closure of the mold 100, which is also able to resist any forces of forming and/or polymerisation and/or hardening of the material that makes up the spacer device and that is inserted into the mold itself. Once the formation and/or the polymerisation and/or the hardening of the spacer device is completed, the mold 100 can be opened, by moving the first half-shell 102 and the second half-shell 104 reciprocally away from each other, causing, possibly in a manual way, the exit and/or the release of the tooth or protrusion element 123a from the engagement seat 123b. In one embodiment, the tooth or protrusion element 123a acts as a hook to constrain temporarily to each other the two half-shells 102, 104 forming the mold 100.

The trigger element 124 has a substantially circular or semi-circular or ergonomic conformation, adapted for the insertion of at least one finger of the operator in charge of forming the spacer device. In this way, the operator can act manually on the trigger element 124. A force may be applied on the trigger element 124 in particular, e.g. pushing or pulling, which may cause the tooth or protrusion element 123a to exit the engagement seat 123b.

As can be seen from the Figures, and as indicated, the tooth or protrusion element 123a and/or the trigger element 124 rises from the second half-shell 104 by a distance such as to make possible the insertion of the tooth or protrusion element 123a into the engagement seat 123b once the mold 100 is closed.

Again as can be seen, as a result of this arrangement the mold according to the present invention has no screws for fixing the first half-shell to the second half-shell and does not even require presses to hold the two half-shells together during the molding step of the spacer device. Both the coupling and the joining of the two half-shells are determined by the presence of the removable constraining structure 122 of the present invention. There are hinge means 130 in at least one version of the invention, for example at the rear in use area 102b2, 104b2 of the first half-shell 102 and/or the second half-shell 104. These hinge means 130 allow reciprocal rotation (on a special fulcrum or centre of rotation) of the second half-shell 104 with respect to the first half-shell 102, bringing them into abutment with each other and thus determining the closure of the mold 100. Conversely, said hinge means 130 also allow the release rotation between said first half-shell 102 and said second half-shell 104, determining the opening of the mold 100. In this step, the first forming surface 108 and the second forming surface 110 move away from each other while in the mold closing step they approach each other, until the cavity 106 and, possibly, the additional cavity 106a, are determined.

Thanks to the presence of the hinge means 130, it is possible to ensure perfect coupling between the first perimeter edge 107 of the first half-shell 102 and the second perimeter edge 109 of the second half-shell 104.

The appropriate bracket exits the first half-shell 102 and/or the second half-shell 104 so that the hinge means 130 are positioned outside the overlapping surfaces of the mold, for example in detail outside the first half-shell 102 at its side wall 102b and/or the rear in use area 102b2 thereof. Similarly, a second special bracket may be present at the second half-shell 104, wherein said second special bracket is outside the second half-shell 104 at its side surface 104b, for example in detail outside its rear in use area 104b2. Moreover, the fulcrum or axis of rotation of the hinge means 130, also by means of the suitable brackets, is positioned "offset" with respect to the plane joining the first forming surface 108 and the second forming surface 110 and/or the first half-shell 102 and the second half-shell 104. This means that the fulcrum or axis of rotation is located on a different plane with respect to the joining plane. The joining plane and the plane on which the rotation axis lies are separated by a distance corresponding to the length, for example, of the special bracket present in the first half-shell 102.

In this way, in at least one version of the invention, the forces of polymerisation and/or hardening of the material that forms the spacer device, forces possibly also caused by the counter-reaction of the compression pressure occasioned by the closure of the mold and that would tend to open the mold, are at least in part absorbed by the structure of the mold itself and, due to the positioning of the fulcrum or axis of rotation of the hinge means 130, are of a lower intensity than if the hinge means 130 were positioned at the plane joining the first half-shell 102 and the second half-shell 104.

Indeed, as mentioned, the first perimeter edge 107 and the second perimeter edge 109 abut against each other when the mold is closed, guaranteeing a substantially sealed closure and maintaining a complete contact and/or a complete adhesion between each other along their entire extension. Thus, the first perimeter edge 107 is in abutment against and/or is matches and/or corresponds to the second perimeter edge 109. In this way, the spacer device resulting from the forming step (and therefore after its extraction from the mold) does not require further finishing or cutting steps of the molding burr. In fact, the perimeter edges 107 and/or 109 act almost like a device that cuts the molding burr directly during the forming step.

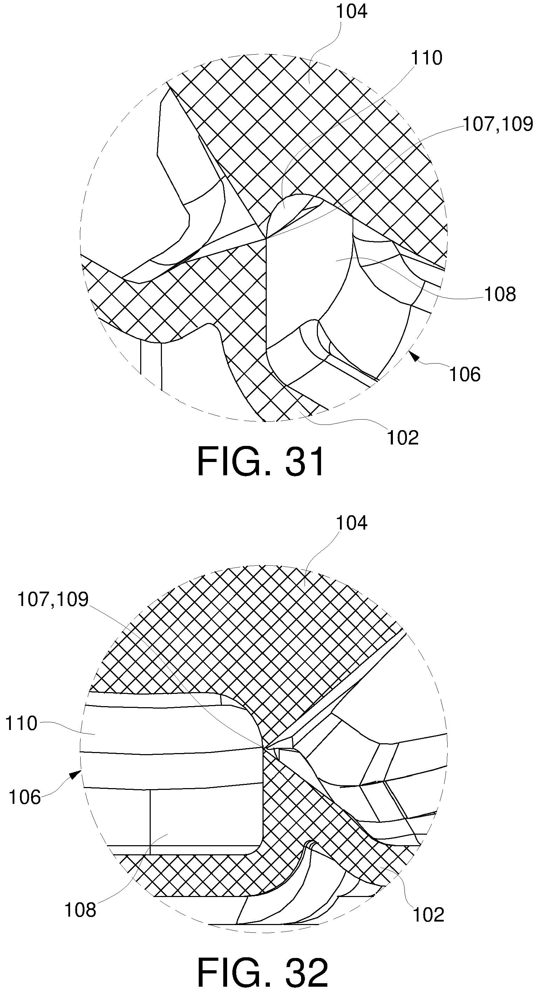

At least one of the first perimeter edge 107 and the second perimeter edge 109, therefore, is an abutting cutting edge. Details of such edges can be found in Figures 31 and 32 (which represent one embodiment of the invention but are also exemplary of the other embodiments, despite the diversity of the forming surfaces of the other versions). Furthermore, when in contact, the perimeter edges 107 and 109 make possible a substantial continuity of the first forming surface 108 with the second forming surface 110. This means that no spaces or gaps are created between the first perimeter edge 107 and the second perimeter edge 109, which could, if present, be invaded by the material constituting the spacer device during the forming step thereof, creating molding burrs that would subsequently have to be eliminated. At the same time, the cavity 106 determined by the first and second forming surfaces 108, 110 corresponds exactly to the shape of the spacer device to be formed, without edges or irregularities that would then require polishing or surface finishing work on the spacer device itself.

In one version of the invention, the two edges have flat abutment surfaces that come into direct contact with each other. These abutment surfaces may be substantially parallel to the support plane of the mold 100 or both have the same inclination, outward or inward, with respect to the cavity 106.

In an alternative version, the flat abutment surfaces of the first perimeter edge 107 and the second perimeter edge 109 are both inclined, one outward and the other inward, with respect to the cavity 106. In this case, too, they manage to leave no gaps with respect to the cavity 106, thus eliminating the formation of molding burrs or reducing them to a minimum, since they are cut during the molding process.

In an alternative version, the first perimeter edge 107 has an abutment surface, which may be flat, for example, while the second perimeter edge 109 has an abutment seat shaped into an area for receiving and/or housing the first perimeter edge 107 (or vice-versa). The above-mentioned results can also be achieved in this way.

In one version of the invention, at least one of such abutment surfaces may, for example, measure 1 mm.

As can be seen, the contact between the first perimeter edge 107 and the second perimeter edge 109 determines the burr-cutting function of these edges.

The minimum support surface present between the two edges 107, 109 (and consequently between the first half-shell 102 and the second half-shell 104) ensures that the force applied in closing (possibly by acting on the handle 112 and/or by means of the removable constraining structure 122) is sufficient to cut and/or divide the material necessary for the formation of the spacer device from the surplus material that escapes into the space outside the cavity 106, beyond the edges 107, 109 themselves.

"Minimum contact surface" means the minimum surface necessary to guarantee the stability of the structure and its ability to withstand the forces applied to it, as well as to guarantee the correct closure of the forming cavity and the correct positioning of the first and second forming surfaces in relation to each other.

The first and the second perimeter edges are adapted to constitute, in use, a single and continuous peripheral edge (transverse or longitudinal) of the resulting spacer device.

In an alternative version, there are no hinge means 130 and first half-shell 102 and second half-shell 104 are moved away from and/or brought closer to each other according to other methods known in the field.

In at least one version of the invention, the mold 100 can also comprise extraction means 132.

The extraction means have the function of extracting the spacer device once it has been formed and once, therefore, the material of which it is composed has hardened. In addition, since they are unidirectional and/or non-return type extraction means, they have a non-return geometry that makes the mold 100 of disposable type.

This aspect is very important because, after having formed a spacer device, the mold might undergo changes or alterations caused precisely by the polymerisation and/or hardening reaction of the material that constitutes the device itself and which could compromise its correct functioning. Such a mold should therefore not be re-used more than once. In the event of re-use, the two half-shells and the other components need to be sterilised and the sterilization step too could cause modifications or alterations that could compromise its correct functioning.

Again, only the use of disposable molds guarantees optimal sterilisation thereof.

As can be seen, for example in Figure 5, which represents an enlarged detail of Figure 4, and in Figure 7, the extraction means 132 comprise a button element 132a including a base 132b and a pressing body 132c1 having a tip or end 132c. The base 132b is opposite the tip or end 132c.

From the version visible in Figure 5, and which may be absent in other versions, during the forming step the tip or end 132c of the pressing body 132c1 is positioned inside the additional cavity 106a and therefore occupies a small part of the space intended for formation of the stem. Accordingly, at the distal end of the stem there may be a small cavity, created precisely by such a tip or end 132c.

The pressing body 132c1 has a substantially pin or peg conformation extending from the base 132b and ending with the tip or end 132c.

The base 132b is placed at the lower face in use 102c of the first half-shell 102, possibly flush with the latter. The tip or end 132b is positioned at the additional cavity 106a, in particular at the most distal area of said additional cavity 106a. In a version in which the additional cavity 106a is not present, the tip or end 132b is placed at the cavity 106.

A space 132d is also envisaged, for example a box-like space the cross-section of which corresponds to the cross-section of the (e.g. circular) base 132b and the height of which corresponds to the stroke of the button element 132a. This space 132d can be opened from at least one side, from two sides in the accompanying Figures.

In use, the operator in charge of the formation of the spacer device, once the latter has been formed, will press, for example manually, on the base 132b of the button element 132a (said base being placed at an opening in turn placed at the space 132d), so that the tip or end 132c presses against the stem 1002 or against the lower face 1008 of the tibial plate 1001 and determines the ejection of the spacer device formed by the mold 100 and/or the cavity 106 of the first half-shell 102.

The height of the space 132d is therefore lower than or equal to the height of the pressing body 132c1 and/or is lower than that of the button element 132a.

As can be seen from the detail of Figure 5, the pressing body 132c1 may have a series of lateral protrusions 132c2 extending externally from the pressing body 132c1 and shaped, for example, like a dovetail.

The button element 132a and/or the pressing body 132c1 and/or the lateral protrusions 132c2 are made of a deformable material, which allows the passage of the tip or end 132c through a special hole 132e present at the additional cavity 106a or the cavity 106 when the button element 132a is pressed to extract the spacer device, but do not allow it to escape or return to position. In this way, the pressing body 132c1 remains inside the additional cavity 106a or the cavity 106, effectively preventing a second or subsequent use of the mold 100.

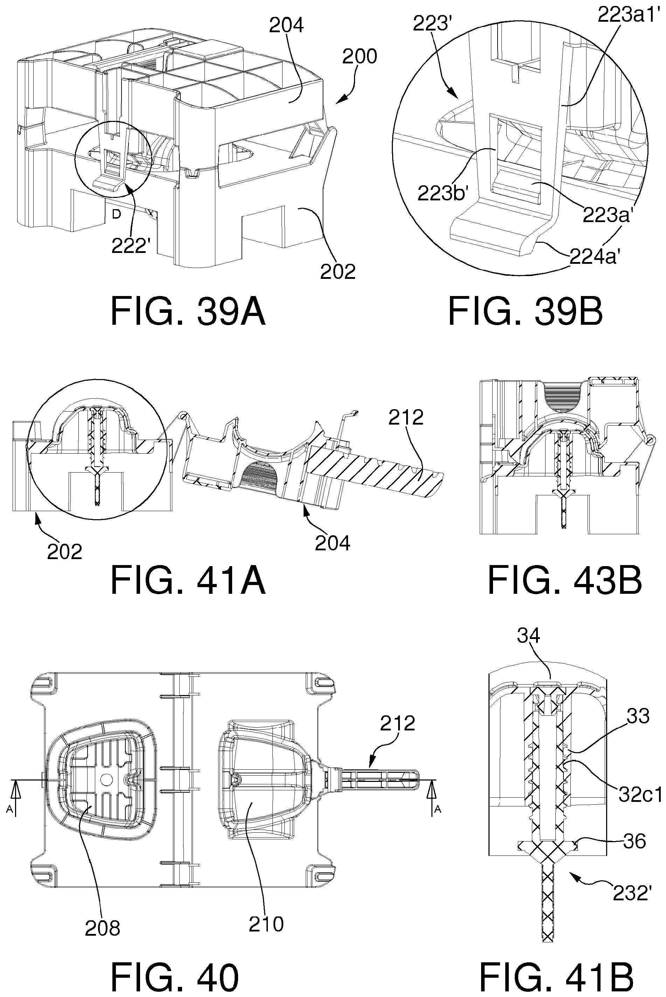

Conversely, with reference to Figures 41B, 44 and 46B to 47B, extraction means 232' may be present. They comprise a pressing body 32c1 comprising a base 32b and a tip or end 32c.

The pressing body 32c1 has a substantially pin or peg conformation and a thread adapted to engage, in a unidirectional manner, with a special seat 33 (possibly corresponding to the space 132d) located at the first half-shell 202, substantially in a central position with respect to the first forming surface 208.

The seat 33 has a nut screw (or further threading) corresponding to the threading of the pressing body 32c1.

The extraction means 232' can also comprise a cap or lid 34 that can be housed and/or positioned at the tip 32c.

Such a cap or lid 34 can be housed, for example snapping-on, in a suitable seat or opening 34a located at the first forming surface 208, in communication with the cavity 206 and the seat 33.

The cap or cover 34 engages with a relative seat present at the tip 32c of the pressing body 32c1.

The extraction means 232' further comprise a handle element 35 capable of being grasped by the operator for the purpose of screwing the extraction means into their seat 33. Overall, the extraction means 232' have a substantially key-shaped conformation.

As can be seen in Figure 41B, when the extraction means 232' is screwed into the seat 33, it comes into contact with the cap or lid 34. While screwing, the pressing body 32c1 presses -- by force of the screwing - against the cap or lid 34; the latter presses against the formed spacer device and causes it to detach from the mold 200 and/or the first half-shell 202.

Between the pressing body 32c1 and the handle 35 there is a discoidal base 36 adapted to comprise at least a pair of teeth 36a made of flexible material. Said teeth 36a are adapted to make it possible to rotate the extraction means 232' but not to allow their unscrewing. In this way, there is additional assurance that the mold is in fact disposable. The teeth 36a are suitable for engaging, for example in rotational screwing mode and/or during screwing of the pressure body 32c1, with respective couplers 36b, located at the seat 33.

Such couplers 36b are adapted to block the unscrewing of the extraction means 232', as they do not allow the passage of the teeth 36a during unscrewing.

For example, the teeth 36a have a C or L shape, so that they deform and can pass over the couplers 36b when screwing in, but block and prevent passage when unscrewing.

The cap or lid 34 is positioned prior to the positioning of the material that will form the spacer device or bone cement and also serves to make the first forming surface 208 substantially continuous.

These extraction means 232' may also be present in other versions of the mold according to the present invention.

Again, in one version of the invention, the second forming surface 110 of the second half-shell 104 has no undercuts. Thus, once molded, the spacer device will remain constrained (albeit temporarily) only to the first forming surface 108 of the first half-shell 102 and to the cavity 106. In this way, it is easier to remove the spacer device formed by the first half shell 102, for example using the extractor means 132.

In a further version, if the second forming surface 110 also has undercuts and/or cavities, suitable extraction means (not illustrated) could also be provided from the second half shell 104 or application of detaching means could be contemplated, at least on the second forming surface 110 (or possibly also on the first forming surface 108), such as to facilitate the detachment of the spacer device after its formation.

Depth gauges 140 may be present at the sides of the mold 100 and at the upper face in use 102a and/or the lower face 104a of the first half-shell 102 and the second half-shell 104, respectively. Said depth gauges 140 are placed, for example, at the corners of the upper face 102a and/or the lower face 104a or at the sides thereof (taking the sides as the surfaces extending between the front in use area 102b1 and the rear in use area 102b2 of the first half-shell 102).

These depth gauges 140 may have complementary shapes between depth gauges present at the first half-shell 102 and depth gauges present in the second half-shell 104 or between depth gauges present at the first half-shell 102 or relative seats present at the second half-shell 104 (or vice-versa), so as to facilitate closure of the mold 100 and maintenance of the correct forming space when the second half-shell 104 is closed onto the first half-shell 102.

The depth gauges 140 may protrude respectively from the upper face 102a and/or the lower face 104a or at least some depth gauges 140 may be recessed into the upper face 102a and/or the lower face 104a.

The depth gauges 140 may preferably be even in number, e.g. two or four or more for each half-shell 102, 104.

In at least one version, the depth gauges 140 can determine the realisation, when the mold is closed, of a space between upper face 102a of the first half-shell 102 and upper face 104a of the second half-shell 104 in which the material that will make the spacer device can possibly flow, if present in excess of the amount required for forming the spacer device. As a result of the presence of the first perimeter edge 107 and the second perimeter edge 109, any excess and leaking material will be eliminated (cut) when the same edges are brought into contact with each other and the two half-shells 102, 104 are brought into abutment and tightened together, for example by means of the removable constraining structure 122. The cut and/or excess material can be deposited in the space created by the fact that the first perimeter edge 107 and the second perimeter edge 109 are raised respectively in relation to the first 102 and the second 104 half-shell, without interfering with the subsequent extraction of the spacer device formed in the mold according to the present invention.

The height of the depth gauges 140 facilitates perfect contact between the first perimeter edge 107 and the second perimeter edge 109 when the mold 100 is in the closed configuration.

When the mold 100 is open, the first perimeter edge 107 and the second perimeter edge 109 constitute, respectively, the access openings for the first half-shell 102 and the second half-shell 104. The material making up the spacer device 1000 can be introduced through such access openings, or at least through the access opening determined by the first perimeter edge 107 of the first half-shell 102.

As a result of the mold according to the present invention, the material making up the spacer device 1000 can have a very high viscosity. In fact, the forces involved in the mold 100 closing step, as well as the specific conformation thereof, also facilitate compression of a very dense and/or viscous material, ensuring that the same flows over the entire area consisting of the cavity 106 and the additional cavity 106a, without leaving empty spaces, thereby obtaining a fully formed spacer device 1000.

In particular, in at least one version of the present invention, the material forming the spacer device is intentionally dense and/or viscous and/or manipulable, so as to limit as much as possible the volumetric shrinkage phenomena that occur during the solidification and/or polymerisation step of the material itself, and which are very pronounced in the case, for example, of cement in a liquid state.

This also facilitates the realisation of the forming operations, in the case in which this material is positioned on both the first and second forming surfaces (when the mold is in open e.g. book-like form).

Thanks to the conformation of the mold 100, in fact, when it is closed, an active pressure is created able to compress and deform the material constituting the spacer device, causing it to flow into all the areas of the cavity 106 and/or the additional cavity 106a, so as to obtain a perfect filling of them by the material in question, with consequent optimal forming of the spacer device in all its parts.

The material constituting the spacer device is supplied to the mold by casting and/or placement on the first forming surface 108 and/or the second forming surface 110, but, as mentioned, not by injection into the mold 100.

In particular, in at least one version of the invention, the above material is positioned at the first forming surface 108. In an alternative version, the material may be positioned at the second forming surface 110 or on both the first forming surface 108 and the second forming surface 110.

With regard to Figures 9 to 15, reference will now be made to a mold 200 for forming the femoral knee spacer device 2000.

Elements identical to the previous embodiment will be indicated with the same reference number increased by one hundred units.

Unless otherwise expressly indicated the opposite, the features and elements described for the previous embodiment may be present and provide the same results also for the mold 200 to which reference will now be made.

The mold 200 is very similar to the mold 100 for forming the tibial knee spacer device. What changes most are the forming surfaces 208 and 210, which in the mold 200 are dedicated to forming the femoral knee spacer device 2000.

The mold 200 thus comprises a first half-shell or lower body 202, comprising a first forming surface 208, and a second half-shell or upper body 204, comprising a second forming surface 210, and a cavity 206 defined between the first half-shell 202 and a second half-shell 204 (or rather between their forming surfaces 208 and 210) when the latter are coupled together.

The first forming surface 208 is defined by a first perimeter edge 207 while the second forming surface 210 is defined by a second perimeter edge 209. The first perimeter edge 207 is adapted to be brought into abutment against the second perimeter edge 209, as disclosed in relation to the first embodiment.

With particular reference to the Figures indicated, the first forming surface 208 is adapted to form the upper face 2008 of the femoral knee spacer device 2000, the latter being illustrated in Figures 16A and 16B, while the second forming surface 210 is adapted to form the lower face 2010 of the spacer device 2000.

The lower face 2010 is adapted in use to be articulated with the tibial knee spacer device (for example, the one illustrated in figures 8A and 8B or a generic tibial knee spacer device) or with the tibial end of a knee joint of a patient while the lower face 2010 is adapted in use to be implanted at the end of the femoral bone of the patient at the knee joint.

Both the first forming surface 208 and the second forming surface 210 have a curved profile. **In** particular, the first forming surface 208 has a convex pattern while the second 210 has a concave pattern.

The cavity 206 substantially determines the formation of the femoral spacer device that has a substantially rectangular or oval or C-shaped conformation in the plan view and a thickness 2004 given by the distance between the upper face 2008 and the lower face 2010 of the spacer device 2000.

A handle 212 is present at the second half-shell 204, possibly removable and possibly equipped with reinforcing elements 212a, completely similar to the handle 112 disclosed in relation to the first embodiment.

The first half-shell 202 comprises at least one upper face 202a, adapted to house the first forming surface 208, and a side wall 202b, while a lower face 202c may or may not be present.

The first forming surface 208 is constituted by a base 208a, convex with respect to the upper face 202a, and a side surface 208b. The side surface 208b extends along the perimeter of the base 208a, from the latter to the first perimeter edge 207. The first perimeter edge 207 is therefore raised with respect to the base 208b and/or the upper face 202a.

In particular, the base 208a may have a series of ribs 215 adapted to determine the formation of respective longitudinal grooves 2015 of the upper face 2008 of the tibial plate 2001.

The upper face 202a is also connected to the first perimeter edge 207 by a raised surface 208c that is substantially inclined or perpendicular to the upper face 202a and, in at least one version of the invention, extends away from the first perimeter edge 207 and/or from the base 208a.

In a substantially similar manner, the second half-shell or upper body 204 comprises at least one lower face 204a, adapted to house, in a substantially central position, the second forming surface 210, a side wall 204b and possibly an upper face 204c.

The second forming surface 210 consists of a base 210a, concave with respect to the lower face 204a. In one version, the base 210a rises from the lower face 204a and the second perimeter edge 209 is therefore raised with respect to the lower face 204a.

In particular, the base 210a may have a substantially smooth surface, although, in at least one version of the invention, it comprises a longitudinal central rib 216 corresponding to a substantially rectangular base recess 2016 present at the lower face 2010 of the femoral spacer device 2000.

The lower face 204a is connected to the second perimeter edge 209 by a raised surface 210c, substantially inclined or perpendicular with respect to the lower face 204a.

At the side wall 202b of the first half-shell 202 and the side wall 204b of the second half-shell 204, it is possible to identify a front in use area 202b1 and 204b1 and a rear in use area 202b2 and 204b2.

At the front area in use 204b1 of the second half-shell 204, a removable constraining structure 222 may be positioned, adapted to be constrained in a removable way with the first half-shell 202.

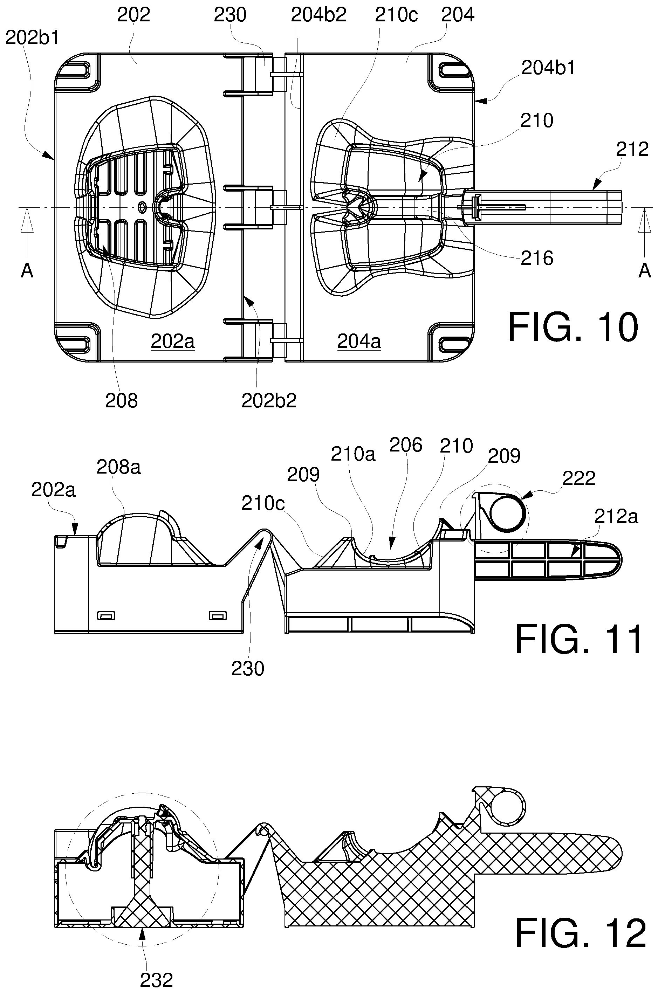

In particular, as illustrated for example in the enlarged detail of Figure 14 (taken from Figure 11), the removable constraining structure 222 comprises, in a manner similar to that described for the first embodiment, coupling means 223, for example snap-on, and possibly a trigger element 224.

The removable coupling means 223 comprise, for example, a tooth or protrusion element 223a, adapted to be engaged in a removable way in a suitable engagement seat 223b placed, for example, at the front in use area 202b1 of the side wall 202b of the first half-shell 202.

There are hinge means 230 in at least one version of the invention, for example at the rear in use area 202b2, 204b2 of the first half-shell 202 and/or the second half-shell 204.

In addition, in at least one version of the invention the mold 200 can also comprise extraction means 232.

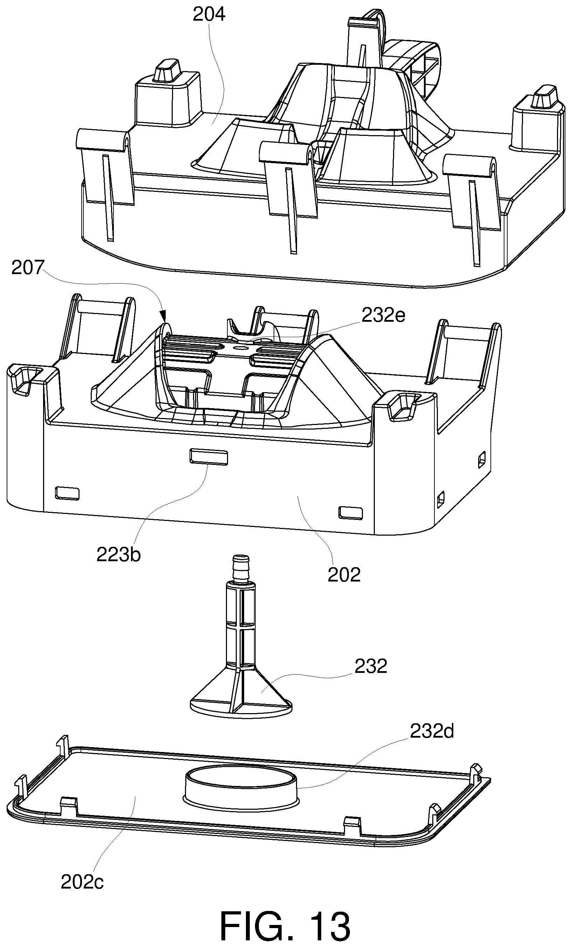

As can be seen, for example in Figure 15, which represents an enlarged detail of Figure 12, and Figure 13, the extraction means 232 comprise a button element 232a comprising a base 232b, a tip or end 232c and a pressing body 232c1.

The base 232b is placed at the lower face 202c of the first half-shell 202, possibly flush with the latter. The tip or end 232b is positioned at the cavity 206, in particular at a central zone thereof, for example; there is then a space 232d. The tip or end 232c presses against the upper surface 2008 of the femoral spacer device 2000 through a suitable hole 232e present at the cavity 206 and/or the first forming surface 208 and causes the spacer device formed by the mold 200 and/or the cavity 206 of the first half-shell 202 to eject.

As can be seen from the detail of Figure 15, the pressing body 232c1 and/or the tip or ends 232c have a series of lateral protrusions 232c2, the disclosure of which has already been made with regard to the first embodiment and which is referred to herein in its entirety.

The extraction means may otherwise be in the version described with the reference number 232'.

Again, the mold 200 may comprise depth gauges 240.

Considering Figures 39A to 47B, reference will now be made to a mold 200 for forming the femoral knee spacer device 2000.

The mold 200 substantially corresponds to that of the previous embodiment, and will therefore not be disclosed any further, except for those aspects that differ from the mold 200.

**In** this version of the mold, the area of manual application of the material that will form the spacer device or bone cement is the first forming surface 208 of the first half shell or lower body 202. Thus, in that version, said material or bone cement for forming the spacer device 2000 is loaded and/or positioned on that surface.

Compared to the previous version of the mold 200, the first forming surface 208 is much more "spacious", as it accommodates substantially all the bone cement necessary for forming the spacer device 2000. Hence the first forming surface 208 is adapted to form, in use, in at least one version, substantially the entire spacer device (meaning in this case the upper face 2008 of the femoral spacer device and substantially its entire thickness 2004).

The second forming surface 210 is therefore only required to form the lower face 2010 of the spacer device 2000.

In this way, it is easier to position the material or cement and subsequently to extract the spacer device already formed.

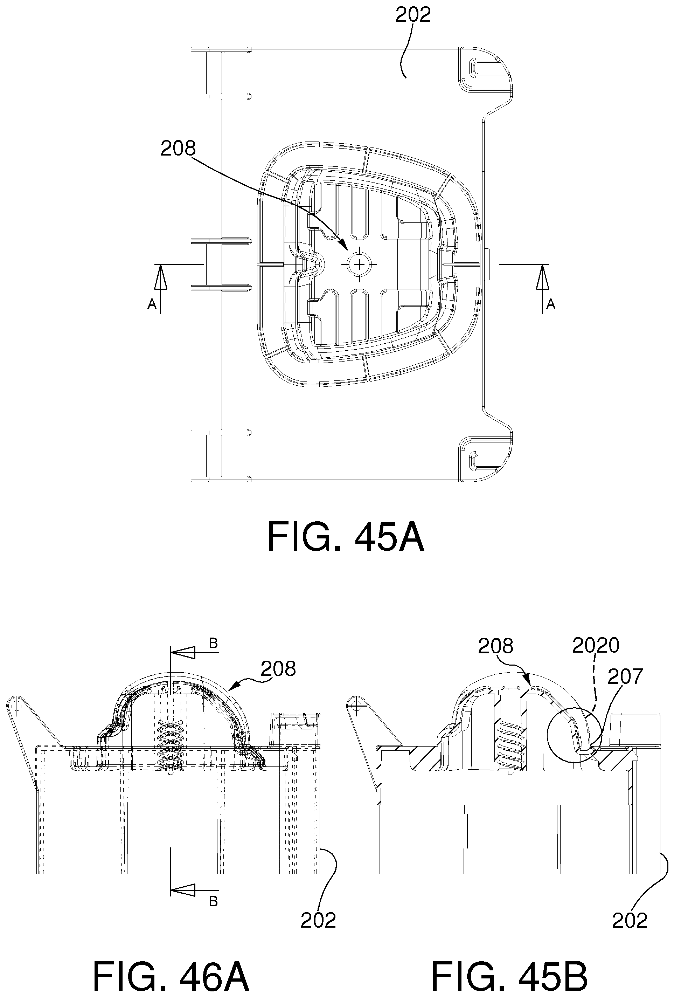

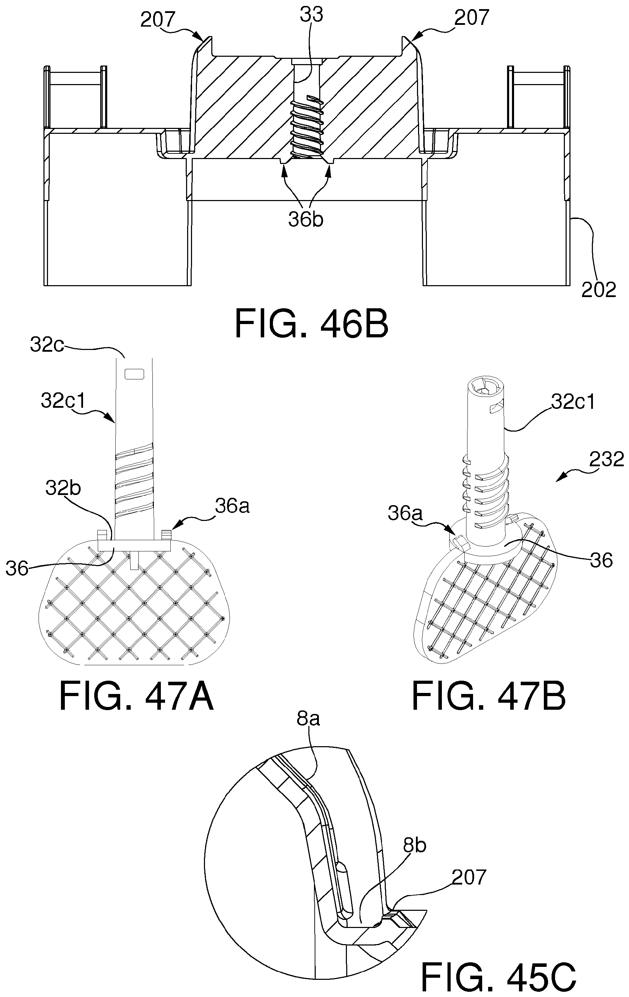

As can be seen, for example from the comparison between Figure 12 and Figure 45C, at the portion of the first forming surface 208 and near the perimeter edge 207 the side surface 208b can have a substantially upward trend towards the cavity 206, for example having a substantially U-shaped conformation (as can be seen in Figure 12) or extending outwards substantially perpendicular to the adjacent base portion 208a (as can be seen in Figure 45C).

This portion is placed at the condylar portion 2020 of the knee joint, with reference to the spacer device 2000, and in more detail with reference to the free ends 2020a of the condylar portion 2020 of the device itself.

In the case of the embodiment illustrated in Figure 45C, it may be easier to remove the molded device.

In this version of the invention (possibly also applicable to the other versions disclosed), as illustrated, for example, in the enlarged detail of Figure 39B, the mold 200 has a removable constraining structure 222' substantially similar to that of the previous versions, comprising removable coupling means 223', for example snap-on means.

The coupling means 223' comprise, for example, a tooth or protrusion element 223a', adapted to be coupled in a removable way in an appropriate engagement seat 223b'. In this case, the tooth is placed at the first half-shell or lower body 202 while the engagement seat 223b' is placed, for example, at the coupling means 223' carried by the second half-shell 204.

In this version, the coupling means 223' comprise a suitable bracket 223a1' adapted to carry the engagement seat 223b' and of a length such as to be able to carry the engagement seat 223b' at the tooth or protrusion element 223a', when the second half-shell 204 is placed over the first half-shell 202 to close the mold 200 and thus start the forming step of the spacer device 2000.

A second end or zone of the bracket 223a1' bears a trigger element 224a'. In this case, the trigger element 224a' has a bracket shape, adapted, for example, to be gripped by a user or adapted to be engaged by a user's finger so as to determine the release of the two half-shells of the mold 200 on completion of the spacer device's formation.

The action on the trigger element 224a' causes the release and/or disengagement of the tooth or protrusion element 223a' from the engagement seat 223b'. Since the trigger element 224a' is in proximity to the handle 212, the operator can, in substantially one-step, grasp the handle 212, release the coupling means 223' using the trigger 224a', and move the second half-shell 204 away from the first half-shell 202 causing the mold 200 to open, for example by rotating these two with respect to each other, again using the handle 212.

A seat 213 for the handle 212 is placed on the side opposite the trigger element 224a'. The trigger element 224a' has a substantially bracket and/or rectangular or semi-circular or ergonomic shape, adapted to engage at least one finger of the operator in charge of forming the spacer device, in order to apply force, for example by pulling or pushing. There may also be a cover element 60, for example shaped as rectangular, square, polygonal, circular sector or circular.

Said cover element 60 is adapted to be housed in a corresponding seat in the upper face 204c of the second half-shell 204.

The cover element 60 is adapted to allow the user's hand positioning, for example resting upon it, for example during mold 200 closing and/or opening steps.

The details of this version can also be applied to other versions disclosed herein, without going beyond the scope of protection of the present invention.

With particular reference to Figures 17 to 21, which illustrate a mold 300 for forming a hip spacer device (the latter illustrated in Figures 22A and 22B), or with particular reference to Figures 23 to 27, which illustrate a mold 400 for forming a shoulder spacer device (the latter illustrated in Figures 28A and 28B), it will be noted that, in such molds, there are respectively a first forming surface 308, 408 of the first half-shell 302, 402, which determines the forming of a first longitudinal half of a hip or shoulder joint spacer device, and a second forming surface 310, 410 of the second half-shell 304, 404, which determines the forming of a second longitudinal half of said spacer device. Therefore, the first and second longitudinal halves of the spacer device include both surfaces in contact with the patient's bone and joint surfaces.

Also in this case, elements identical to the previous embodiment will be indicated with the same reference number respectively increased by one hundred units.

Unless otherwise expressly indicated, the features and elements described for the previous embodiment may be present and provide the same results also for the molds 300 and 400 to which reference will now be made.

The first forming surface 308, 408 comprises a (first) stem portion 308a, 408a, a (first) head portion 308b, 408b, and a (first) connection portion 308c, 408c between the stem portion and the head portion.

Similarly, the second forming surface 310, 410 comprises a (second) stem portion 310a, 410a, a (second) head portion 310b, 410b and a (second) connection portion 310c, 410c between the stem portion and the head portion. Each portion of the first forming surface 308, 408 is adapted to rest against, and at, the respective portion in the second forming surface 310, 410. A cavity 306, 406 is enclosed between the first forming surface 308, 408 and the second forming surface 310, 410. Thus, stem portion 308a, 408a, head portion 308b, 408b and connection portion 308c, 408c are connected to each other in fluid communication; likewise, stem portion 310a, 410a, head portion 310b, 410b and connection portion 310c, 410c are connected to each other in fluid communication.

The cavity 306, 406 determines the forming of the hip 3000 or shoulder 4000 spacer device, each comprising a stem 3001, 4001, a head 3002, 4002 and a connecting neck 3003, 4003. The stem 3001, 4001 may be of different lengths, as illustrated, for example, in Figures 22A, 22B and 28A and 28B.

In at least one version, furthermore, the head 3002, 4002 has a hemispherical conformation. Accordingly, stem portion 308a, 408a, 310a, 310b and head portion 308b, 408b, 310b, 410b are of a length or conformation corresponding to that of the device to be obtained.

In this version of the invention, the first half-shell 302, 402 and the second half-shell 304, 404 may constitute two halves of the mold 300, 400 respectively and thus mirror each other. In this version, the first half-shell 302, 402 and the second half-shell 304, 404 are thus symmetrical with respect to an axis of union of the first half-shell and/or the second half-shell or with respect to a plane parallel to the upper face of the first half-shell and/or the lower face of the second half-shell.

Thus, the first half-shell or lower body 302, 402 comprises a first forming surface 308, 408, while the second half-shell or upper body 304, 404 comprises a second forming surface 310, 410.

The first forming surface 308, 408 is defined by a first perimeter edge 307, 407, while the second forming surface 310, 410 is defined by a second perimeter edge 309, 409; the first perimeter edge is adapted to be brought into abutment against the second perimeter edge, as disclosed in relation to the first embodiment.

At the second half-shell 304, 404 there is a handle 312, 412, possibly removable and possibly provided with reinforcing elements 312a, 412a, completely similar to the handle 112 described for the first embodiment.

The first half-shell 302, 402 comprises at least one upper face 302a, 402a adapted to house the first forming surface 308, 408 and a side wall 302b, 402b, while a lower face 302c, 402c may or may not be present. Similarly, the second half-shell 304, 404 comprises at least one upper face 304a, 404a adapted to house the second forming surface 310, 410 and a side wall 304b, 404b, while a lower face 304c, 404c may or may not be present. The first forming surface 308, 408 and the second forming surface 310, 410 consist of concave surfaces with respect to top face 302a, 402a and to top face 304a, 404a of the first half-shell 302, 402 and the second half-shell 304, 404.

However, between the first forming surface 308, 408 and the first perimeter edge 307, 407 and between the second forming surface 310, 410 and the second perimeter edge 309, 409 there may be a side surface 308e, 408e, 310e and 410e extending along the perimeter of the respective surface, from the latter to the first perimeter edge and to the second perimeter edge. The first perimeter edge 307, 407 is therefore raised with respect to the upper face 302a, 402a of the first half-shell 302, 402; the second perimeter edge 309, 409 is thus raised with respect to the upper face 304a, 404a of the second half-shell 304, 404. Furthermore, the upper face 302a, 402a of the first half-shell 302, 402 is connected to the first perimeter edge 307, 407 by a raised wall 308d, 408d, which is substantially inclined or perpendicular with respect to the upper face 302a, 402a and, in at least one version of the invention, extends away from the first perimeter edge 307, 407.

Similarly, the upper face 304a, 404a of the second half-shell 304, 404 is connected to the second perimeter edge 309, 409 by a raised wall 310d, 410d, which is substantially inclined or perpendicular with respect to the upper face 304a, 404a and, in at least one version of the invention, extends away from the second perimeter edge 309, 409.

At the side wall 302b, 402b of the first half-shell 302, 402 and the side wall 304b, 404b of the second half-shell 304, 404, it is possible to identify a front in use area 302b1, 402b1 and 304b1, 404b1 and a rear in use area 302b2, 402b2 and 304b2, 402b2.

A removable constraining structure 322, 422 adapted to be constrained in a removable way with the first half-shell 302, 402 may be positioned at the front in use area 304b1, 404b1 of the second half-shell 304, 404.

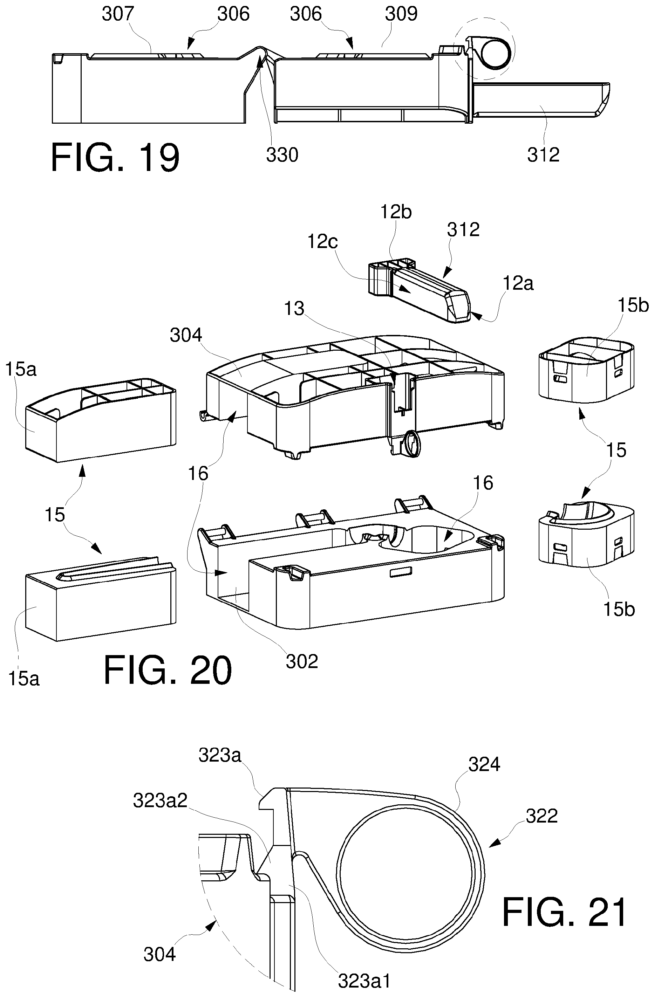

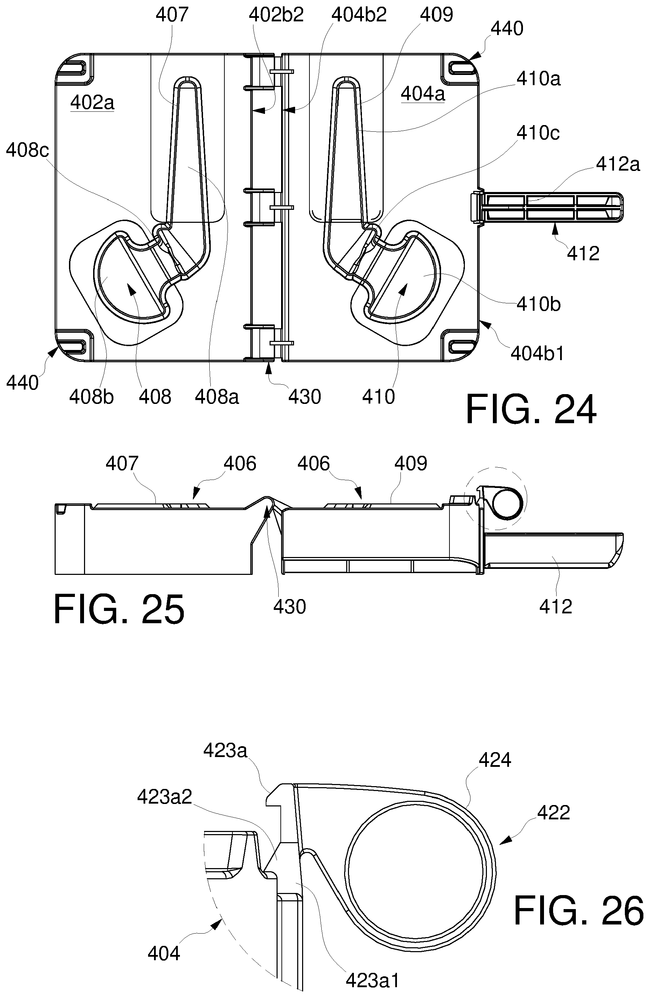

In particular, and as illustrated, for example, in the enlarged detail of Figure 21 (taken from Figure 19) and Figure 26 (taken from Figure 25), the removable constraining structure 322, 422 comprises, in a manner similar to that disclosed in relation to the first embodiment, the coupling means 323, 423, for example snap-on, and possibly a trigger element 324, 424.

The removable coupling means 323, 423 comprise, for example, a tooth or protrusion element 323a, 423a adapted to be coupled in a removable way in a suitable engagement seat 323b, 423b placed, for example, at the front in use area 302b1, 402b1 of the side wall 302b, 402b of the first half-shell 302, 402.

In at least one version of the invention, hinge means 330, 430 are present, for example at the rear in use area 302b2, 304b2, 402b2, 404b2 of the first half-shell 302, 402 and/or the second half-shell 304, 404.

In addition, in at least one version of the invention the mold 300, 400 can also comprise extraction means (not shown in the Figures). Said extraction means may have the same conformation already disclosed for the other embodiments and act without distinction on the first forming surface 308, 408 and/or the second forming surface 310, 410, possibly also on several points thereof.

Also in these embodiments, the mold 300, 400 can include depth gauges 340, 440.

As can be seen, for example in Figure 20 for the mold 300 and in Figure 27 for the mold 400, but also applicable to the molds 100 and 200 disclosed above, the handle 112, 212, 312, 412 can comprise a first end 12a, distal from the second half-shell 104, 204, 304, 404, and a second end 12b, proximal to the second half-shell 104, 204, 304, 404, as well as an elongated body 12c, extending from the first end 12a to the second end 12b.

In so far as this handle is removable, the second end 12b has a conformation corresponding to that of a specific housing seat 13. The housing seat 13 is arranged in the second half-shell 104, 204, 304, 404, in particular at its upper face 104c, 204c, 304c, 404c, for example.

In at least one version of the invention, the second end 12b is of a width and/or a thickness greater than the width and thickness of the elongated body 12c. In addition, the second end 12b may have a width and/or thickness that is greater than the width and thickness of the first end 12a.

Similarly, the housing seat 13 will be of a width and/or thickness corresponding to that of the second end 12b.

Thanks to the insertion of the second end 12b into the housing seat 13, the handle 112, 212, 312, 412 will be mounted in position, so that the operator in charge of forming the spacer device can act on it and then apply the necessary force to close the mold 100, 200, 300, 400 by bringing the first half shell 102, 202, 302, 402 and the second half shell 104, 204, 304, 404 into abutment with each other, and then the first perimeter edge 107, 207, 307, 407 onto the second perimeter edge 109, 209, 309, 409.

Thanks to this expedient, when the mold is not in use, its overall size is significantly reduced, with a positive effect on the size of the packaging and on the mold's storage and transport.

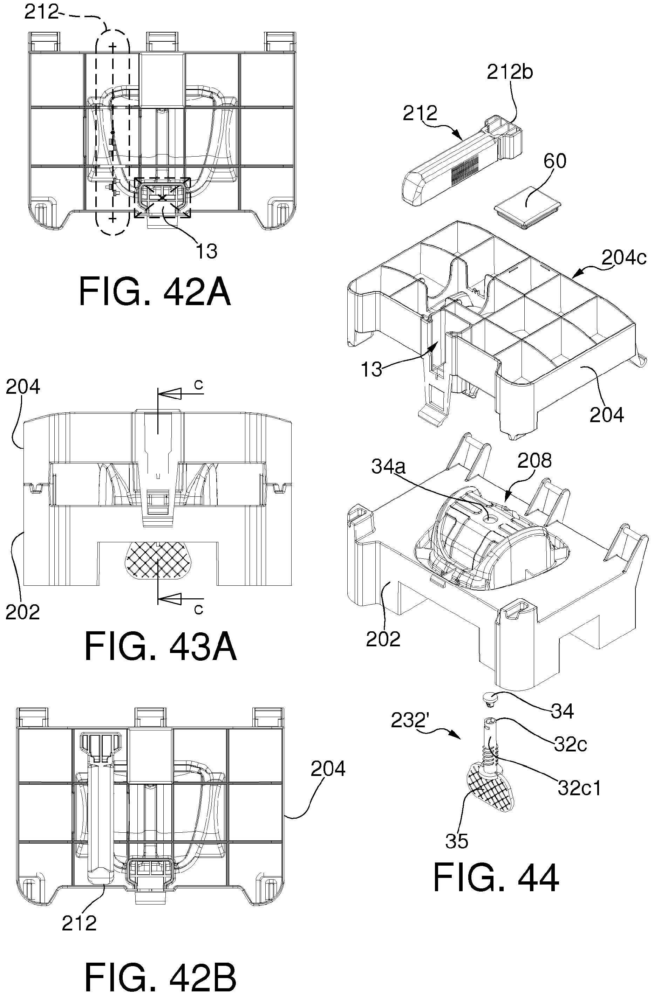

The position of the handle 212 when not in use is shown in Figures 42B and 42A, respectively (in Figure 42A the carrying position of the handle is shown with a dashed line, and in Figure 42B the handle 212 is shown in this carrying position).

**In** a still further version of the invention, illustrated, for example, in Figures 20 and 27, the mold 100, 200, 300, 400 may feature a series of interchangeable inserts 15. Said interchangeable inserts 15 are placed at respectively at least one of first forming surface 108, 208, 308, 408 and second forming surface 110, 210, 310, 410. More specifically, said interchangeable inserts 15, which are removable in at least one version of the invention, comprise, on one face thereof, at least a portion of at least one of first forming surface 108, 208, 308, 408 and second forming surface 110, 210, 310, 410. These interchangeable inserts 15 are used to form spacer devices of different sizes in a single mold.

The interchangeable inserts 15 are foreseen with forming surfaces of different sizes, for example very small, small, medium, large or very large (or only some of these), so as to determine the formation of a spacer device of very small, small, medium, large or very large size (or only some of these).

In this way, the first half-shell and/or the second half-shell acts as an insert holder, provided with a seat 16 for housing the interchangeable inserts 15. The seat 16 and the overall structure of the insert 15 always have the same dimensions, so that each insert 15 can be accommodated in the corresponding seat 16. What varies is the size of the forming surface carried by said inserts 15.

The first half-shell and/or the second half-shell is therefore "universal", in the sense that it has a seat 16 compatible with all interchangeable inserts 15. Furthermore, the elements comprising the mold, all of which are on the first and/or second half-shell, namely, the handle, the removable constraining structure, the hinge means and the extraction means (when all or only some of these elements are present), as well as any centring and other mechanisms, are provided only once, precisely on the first half-shell and/or on the second half-shell, but can be exploited by all the interchangeable inserts and thus to form spacer devices of all necessary or desired sizes.

For the correct functioning of the mold, once the desired size for the spacer device has been selected the corresponding interchangeable insert 15 is selected and positioned on the seat 16 adapted for its housing. Specifically, the interchangeable insert can be fixed permanently to the first half-shell and/or the second half-shell or it can be fixed in a removable way. In the latter way, after forming a spacer device of a certain size by means of an interchangeable insert 15, it is possible to eliminate the insert already used but to use the first half-shell and/or the second half-shell for forming a subsequent spacer device without losing the characteristics of perfect shape and perfect stability that guarantee a completely disposable mold. In this case, only the inserts 15 are disposable.

As seen for the molds 300 and 400, the interchangeable inserts 15 may include a set of stem inserts 15a and a set of head inserts 15b. In this way, each size of stem portion forming surface 310a, 410a can be variously coupled with each size of head portion forming surface 310b, 410b, and vice-versa. Naturally, in such case the seat 16 will comprise a first zone for housing the stem insert 15a and a second zone for housing the head insert 15b. In such molds, the fitting portion 310c may be fixed (e.g. made in one piece with the first half-shell and/or with the second half-shell and/or unique for each size of stem insert or head insert) or may also be provided on a suitable insert.

Again, it is possible for only the stem insert 15a or only the head insert 15b to be provided in the mold, thus only allowing the size of that portion of the spacer device corresponding to the insert to be varied.

Furthermore, stems of various lengths can be provided in the stem insert 15a, while in the head insert 15b different diameters of the head or also different configurations of the same (hemispherical, spherical, spherical cap, and the like) can be provided.

The spacer device according to the present invention, with particular reference to the hip 3000 and shoulder 4000 devices, but also desirable for the tibial 1000 and/or femoral 2000 knee devices, may comprise at least one inner core, in metallic material, for example. The function of this internal core is to strengthen the structure of the spacer device and thus enable it to withstand more intense loads or stresses during its use.

When present, said inner core is positioned at the first and/or the second forming surface. It can be held in position, for example, by the application of special supports or spacers, for example in the form of a yoke, which, at the end of the forming phase, allow it to be in a substantially central internal position within the spacer device. These supports or spacers can be made of the same material of which the spacer device will be made (but already polymerised and/or solidified), so as to join intimately with that material when molding is completed.

Again, the first and/or second forming surface comprises a finish such that the resulting spacer device has a smooth surface, almost glossy, for example, free of imperfections or molding burrs, substantially free of roughness and irregularities. In this way, the forming step of the spacer device can be unique in the sense that a subsequent polishing step of the resulting spacer device is not necessary. As already mentioned, excesses of material emerge from the first and second perimeter edges during the closing step of the first half-shell and the second half-shell and are cut from these edges so as to form an already finished spacer device, with no visible demarcation lines between the areas formed by the first half-shell with respect to the areas formed by the second half-shell.

In addition, these edges pass through the articulating surface of the head of the respective spacer device, but their burr-cutting function is so effective that they do not compromise the articulation of the spacer device in its joint seat.

As noted above, the mold according to the present invention solves the above-mentioned drawbacks in that it has a solid structure, possibly with a simple hinge, possibly with inserts that can be combined to obtain different versions of the molded spacer device, in which the perimeter edges of the abutment and/or junction of the two half-shells have a sharp-edged joint, as a result of which the forming and/or molding operation is of a simplicity never achieved with the molds of the known type.

By means of the mold according to the present invention, it is also possible to ensure a substantially hermetically sealed and constant closure throughout the hardening and/or polymerisation time of the material constituting the spacer device.

The rigidity of the molds according to the present invention, and of any of their inserts, also ensures the non-deformability of the mold and therefore the correct molding of the relative spacer device.

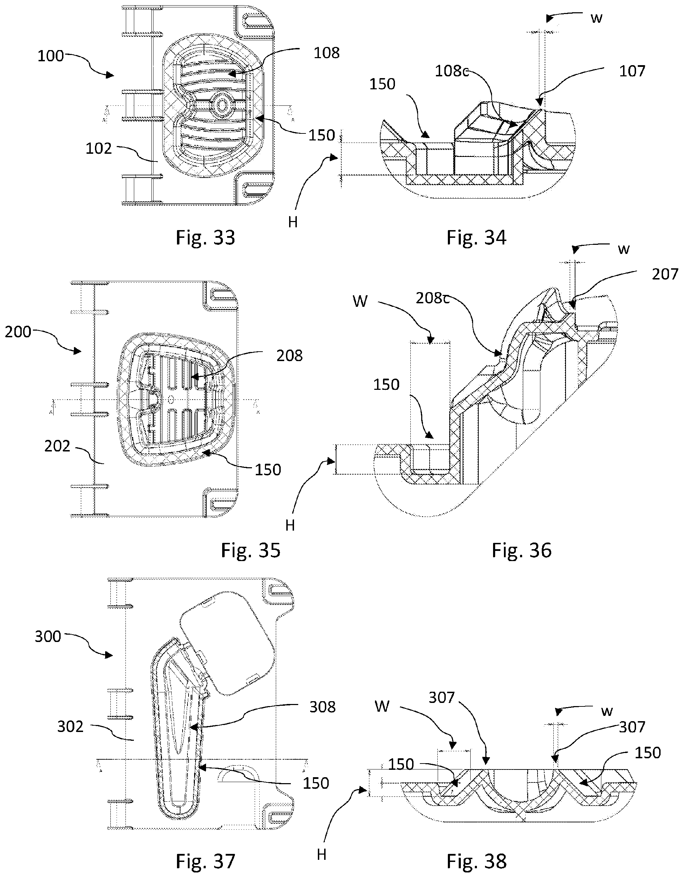

According to a further version illustrated in Figures 33 to 38, the mold 100, 200, 300, 400 may comprise at least one channel 150. Said channel 150 is positioned adjacent to at least one between the first perimeter edge 107, 207, 307, 407 and the second perimeter edge 109, 209, 309, 409 and/or at least one between the first forming surface 108, 208, 308, 408 and the second forming surface 110, 210, 310, 410.

The term "adjacent" means that the channel 150 departs directly from the first or second perimeter edge or is of some distance, albeit small, from that perimeter edge. The same applies to the first or the second forming surface.

For example, as can be seen in Figures 34 and 36, the channel 150 departs from the first raised wall 108c, 208c.

This channel 150 is a kind of gutter. Its function, indeed, is to receive in use the excess material forming the spacer device.

The channel 150 has a trend corresponding substantially to that of the first perimeter edge 107, 207, 307, 407 and/or the second perimeter edge 109, 209, 309, 409 and/or the first and/or the second forming surface and/or a part thereof.

According to one version, the channel 150 is positioned only at the first half-shell or lower body 102, 202, 302, 402.

According to a further version especially dedicated to the mold for forming a hip or shoulder spacer device, the channel 150 can only be positioned at the first stem portion and/or the second stem portion.

If necessary, the channel 150 may also be placed adjacent to or along the first connecting portion and/or the second connecting portion.

Alternatively, for the mold for forming a hip or shoulder spacer device, the channel 150 may also be placed at (adjacent to and/or along) the entire forming surface.

As mentioned above, this channel 150 is adapted to collect the excess cement or in any case the excess material used for forming the spacer device. In this version, indeed, during closure of the mold the excess material exits or overflows from the cavity formed by the first and second forming surfaces and flows or drips into this channel 150, where it continues normal polymerisation.

Furthermore, the channel 150 may have a surface, at least at the bottom, which is knurled and/or has a certain roughness or texture or finish.

In this way, said surface which is knurled and/or has a certain roughness or texture or finish, determines strong adhesion to the cement or to the excess material that flows into it after polymerisation.

In this way, when the mold opens, the channel 150 and/or its adhesion to the cement or excess material aids in the detachment of the excess material flowing precisely into the channel, thereby separating the resulting spacer device both from such excess material and from the forming surface itself.

In this way, too, the spacer device obtained is free of molding burrs, which remain attached to the mold and in particular to the channel 150.

The channel 150 thus acts almost like a "trap" for the excess material.

As can be seen from the accompanying Figures, the channel 150 has a cavity in recess with respect to the surface of the first half-shell and/or the second half-shell, with a wall substantially perpendicular or inclined with respect to the latter.

From a dimensional point of view, the channel 150 can have a width W of about 8 mm or between 6 mm and 10 mm and/or a height or depth H of about 5 mm or 6 mm or between 4 mm and 8 mm.

As can be seen, for example in Figure 38, the height H of the channel 150 may be given by a first section and a second section, wherein the first section is formed by the distance between the upper face 102a, 202a, 302a, 402a of the first half-shell and the deepest point of the channel, while the second section is given by the distance between the upper face 102a, 202a, 302a, 402a of the first half-shell and the outermost or highest point of the channel 150. In one version of the invention, the first and the second section are of the same size, for example about 3 mm.

The outermost or highest point of the channel 150 may be equal to the height (from the upper face of the first half-shell) of the perimeter edge 307, 407. A similar situation may occur if a channel 150 is also present at the second half-shell.

The difference between this version of the invention and previous versions, in which there may have been a space or gap outside the forming cavity and at the first and/or second perimeter edge into which the surplus bone cement could be discharged, is that the channel 150 forms a containment chamber for the excess material in which it is trapped. In addition, its surface (at least the bottom one), which is knurled and/or has a certain roughness or texture or finish, increases the adhesion of said excess material and facilitates its detachment, when hardened and/or polymerised, from the spacer device formed.

Once again, the device formed is substantially smooth and free of machining burrs, and therefore does not require any finishing steps.

In at least one version of the invention, the contact surface between the first perimeter edge and the second perimeter edge has a measurement w of 1 mm or of between 0.5 mm and 2 mm.

As can be seen from the present disclosure, this invention also relates to a method for forming a spacer device for replacing a joint prosthesis comprising the following steps:
providing a mold 100, 200, 300, 400 comprising a first half-shell or lower body 102, 202, 302, 402 comprising a first forming surface 108, 208, 308, 408 delimited by a first perimeter edge 107, 207, 307, 407 and intended to shape at least a first portion of the spacer device, and
a second half-shell or upper body 104, 204, 304, 404 comprising a second forming surface 110, 210, 310, 410 delimited by a second perimeter edge 109, 209, 309, 409 and intended to shape at least a second portion of the spacer device,
providing a material constituting the spacer device and positioning and/or casting the material into the first forming surface 108, 208, 308, 408 and/or the second forming surface 110, 210, 310, 410,
coupling the first half-shell or lower body 102, 202, 302, 402 and the second half-shell or upper body 104, 204, 304, 404,
bringing the first perimeter edge 107, 207, 307, 407 into abutment against the second perimeter edge 109, 209, 309, 409, so as to close the mold and delimit between them, at the first forming surface 108, 208, 308, 408 and the second forming surface 110, 210, 310, 410, a cavity 106, 206, 306, 406 corresponding to the external configuration of the spacer device,
waiting a time for the material to polymerise and/or harden and form the spacer device,
wherein during the step of forming, at least one between the first perimeter edge 107, 207, 307, 407 and the second perimeter edge 109, 209, 309, 409 cuts and/or eliminates any burrs determined by the forming of the spacer device and/or
wherein during the forming step, the forming material of the spacer device flows and/or drips into a channel 150 adjacent to at least one between the first perimeter edge 107, 207, 307, 407 and the second perimeter edge 109, 209, 309, 409 and/or at least one between the first forming surface 108, 208, 308, 408 and the second forming surface 110, 210, 310, 410 and adheres to and/or in the channel 150, so as to eliminate any burrs determined by the forming of the spacer device.

In at least one version of the invention, the step of positioning and/or casting the material takes place manually, which is to say, for example, without the use of instruments such as material injection tools.

The coupling step comprises operating a handle 112, 212, 312, 412, for example manually, and/or operating a removable constraining structure 122, 222, 322, 422, present in the second half shell 104, 204, 304, 404 and/or the first half shell 102, 202, 302, 402, and constraining in a removable way the first half shell 102, 202, 302, 402 with the second half shell 104, 204, 304, 404.

The operating step comprises actuating coupling means 123, 223, 323, 423, for example snap-on, of a removable type of the removable constraining structure 122, 222, 322, 422 and/or carrying a tooth or protrusion element 123a, 223a, 323a, 423a present at the second half-shell 104, 204, 304, 404 at a suitable engagement seat 123b, 223b, 323b, 423b, having a conformation corresponding and/or complementary to that of the tooth or protrusion element 123a, 223a, 323a, 423a, realised in the first half-shell 102, 202, 302, 402 or vice-versa and engaging, for example by snapping, the tooth or protrusion element 123a, 223a, 323a, 423a in said engagement seat 123b, 223b, 323b, 423b.

The method further comprises a step of extracting the formed spacer device from the mold and such step comprises pulling the first half-shell 102, 202, 302, 402 and the second half-shell 104, 204, 304, 404 away from each other, releasing the coupling means 123, 223, 323, 423 by disengaging the tooth or protrusion element 123a, 223a, 323a, 423a from the engagement seat 123b, 223b, 323b, 423b, and/or acting on a trigger element 124, 224, 324, 424, 224a' of the removable constraining structure 122, 222, 322, 422 and/or on the handle 112, 212, 312, 412.

The step of extracting comprises actuating unidirectional-type extraction means 132, 232, 332, 432, 232', suitable for extracting the spacer device after it has been formed and making the mold disposable, and said actuating step comprises pressing a base 132b, 232b, 332b, 432b of a button element 132a, 232a, 332a, 432a so that a tip or end 132c, 232c, 332c, 432c of a pressing body 132c1, 232c1, 332c1, 432c1 of the button element 132a, 232a, 332a, 432a, wherein the tip or end 132c, 232c, 332c, 432c faces the cavity 106, 206, 306, 406 and/or the additional cavity 106a, pushes the spacer device out of the cavity 106, 206, 306, 406 and/or the additional cavity 106a, or wherein the step of actuating comprises screwing a pressing body 32c1 into a suitable seat 33 realised in the first half-shell 102, 202, 302, 402 such that a tip or end 32c of the pressing body 32c1 that faces into the cavity 106, 206, 306, 406 and/or the additional cavity 106a, pushes the spacer device out of the cavity 106, 206, 306, 406 and/or the additional cavity 106a. The molds described above are subject to numerous modifications and variations within the scope of protection of the following claims.

## Claims

1. Mold (100, 200, 300, 400) for forming a spacer device for replacing a joint prosthesis, such as a tibial knee spacer device, suitable to be implanted in use at the tibial bone of the knee joint, a femoral knee spacer device, suitable to be implanted in use at the femoral bone of the knee joint, a hip spacer device, suitable to be implanted in use at the femoral bone of the hip joint, or a shoulder spacer device, suitable to be implanted in use at the shoulder joint, comprising:
a first half-shell or lower body (102, 202, 302, 402) comprising a first forming surface (108, 208, 308, 408) delimited by a first perimeter edge (107, 207, 307, 407) and intended to mould at least a first portion of said spacer device, and
a second half-shell or upper body (104, 204, 304, 404) comprising a second forming surface (110, 210, 310, 410) delimited by a second perimeter edge (109, 209, 309, 409) and intended to mould at least a second portion of said spacer device,
said first half-shell or lower body (102, 202, 302, 402) and said second half-shell or upper body (104, 204, 304, 404) being able to be coupled, in use, in a removable way, so as to define for said mold (100, 200, 300, 400) a closed configuration and delimit between them, at said first forming surface (108, 208, 308, 408) and said second forming surface (110, 210, 310, 410), as well as at said first perimeter edge (107, 207, 307, 407) and said second perimeter edge (109, 209, 309, 409), a cavity (106, 206, 306, 406) corresponding to the external configuration of the spacer device,
said first perimeter edge (107, 207, 307, 407) being able to abut and come into contact, in use, with said second perimeter edge (109, 209, 309, 409),
**characterized by the fact that**
at least one of said first perimeter edge (107, 207, 307, 407) and said second perimeter edge (109, 209, 309, 409) is an abutting cutting edge, so as to cut and/or eliminate in use any moulding burrs determined by the forming of said spacer device when said mold is in a closed configuration, **by the fact that** at least one of said first perimeter edge (107, 207, 307, 407) and said second perimeter edge (109, 209, 309, 409) is raised with respect to said first half-shell or lower body (102, 202, 302, 402) and to said second half-shell or upper body (104, 204, 304, 404), respectively **and by the fact that** said first perimeter edge (107, 207, 307, 407) is continuous along all said first forming surface (108, 208, 308, 408) and said second perimeter edge (109, 209, 309, 409) is continuous along all said second forming surface (110, 210, 310, 410).

2. Mold according to claim 1, wherein said mold (100, 200, 300, 400) comprises at least one channel (150) adjacent to at least one of said first perimeter edge (107, 207, 307, 407) and said second perimeter edge (109, 209, 309, 409) and to at least one of said first forming surface (108, 208, 308, 408) and said second forming surface (110, 210, 310, 410), wherein said channel is adapted to receive in use forming material in excess for said spacer device.

3. Mold according to claim 1 or 2, wherein said first perimeter edge (107, 207, 307, 407) abuts against or mates with or corresponds to said second perimeter edge (109, 209, 309, 409) and/or wherein said first perimeter edge (107, 207, 307, 407) has a minimum contact surface with said second perimeter edge (109, 209, 309, 409) and/or wherein said first perimeter edge (107, 207, 307, 407) and said second perimeter edge (109, 209, 309, 409) have flat abutting surfaces which come into direct contact one with the other, wherein said abutting surfaces are substantially parallel to a support plane for said mold (100, 200, 300, 400) or both having the same inclination, towards the outside or towards the inside, with respect to said cavity (106, 206, 306, 406), or wherein said abutment surfaces are both inclined, one towards the outside and the other towards the inside, with respect to said cavity (106, 206, 306, 406), or wherein one between said first perimeter edge (107, 207, 307, 407) and said second perimeter edge (109, 209, 309, 409) has an abutment surface, for example substantially flat, while the other between said first perimeter edge (107, 207, 307, 407) and said second perimeter edge (109, 209, 309, 409) has an abutment seat shaped as an acceptance or housing area for said abutment surface.

4. Mold according to any one of the preceding claims, comprising a removable constraining structure (122, 222, 322, 422, 222'), present in said second half-shell (104, 204, 304, 404) and/or in said first half-shell (102, 202, 302, 402), adapted to removably constrain said first half-shell (102, 202, 302, 402) with said second half-shell (104, 204, 304, 404), wherein said removable constraining structure (122, 222, 322, 422, 222') is equipped with coupling means (123, 223, 323, 423, 223'), for example snap-on, which comprise for example a tooth or protrusion element (123a, 223a, 323a, 423a, 223a'), present at said second half-shell (104, 204, 304, 404) adapted to engage in a removable way in a suitable engagement seat (123b, 223b, 323b, 423b, 223b'), having a conformation corresponding or complementary to that of said tooth or protrusion element (123a, 223a, 323a, 423a, 223a'), made in said first half-shell (102, 202, 302, 402) or vice versa.

5. Mold according to any one of the preceding claims, comprising a handle (112, 212, 312, 412) adapted to be gripped for bringing said second half-shell or upper body (104, 204, 304, 404) in abutment against said first half-shell or lower body (102, 202, 302, 402) and to apply a force to compress the material that will constitute the spacer device, wherein said handle (112, 212, 312, 412) is placed at said second half-shell (104, 204, 304, 404) and/or is removable.

6. Mold according to claim 4 and 5, wherein said removable constraining structure (122, 222, 322, 422, 222') comprises a trigger element (124, 224, 324, 424, 224a'), wherein said trigger element (124, 224, 324, 424, 224a') is placed at said handle (112, 212, 312, 412), said trigger element (124, 224, 324, 424, 224a') being able to be acted during use by an operator, once the forming step of said spacer device has been completed, and therefore after having waited a predetermined time for the hardening of the material that constitutes said spacer device, to determine the release and/or uncoupling of said tooth or protrusion element (123a, 223a, 323a, 423a, 223a') from said engagement seat (123b, 223b, 323b, 423b, 223b').

7. Mold according to claim 5 or 6, wherein said handle (112, 212, 312, 412) has a substantially elongated and possibly ergonomic conformation, with a substantially rectangular longitudinal section, with rounded edges and with a substantially rectangular, square, circular, triangular cross section, etc., and/or wherein said handle (112, 212, 312, 412) internally comprises reinforcing elements (112a, 212a, 312a, 412a), for example some ribs.

8. Mold according to any one of the preceding claims, wherein said cavity (106, 206) and said first forming surface (108, 208) and/or said second forming surface (110, 210) has a plan conformation substantially rectangular, oval or C shaped, corresponding to that which the resulting tibial spacer device or femoral spacer device will have, and/or wherein said mold (100) comprises an additional cavity (106a), adapted in use to form a stem (1002) of said tibial spacer device, and placed at said first forming surface (108), wherein said additional cavity (106a) and said cavity (106) are contiguous to each other and in fluid connection.

9. Mold according to any one of the preceding claims, wherein said first half-shell (102, 202, 302, 402) and/or said second half-shell (104, 204, 304, 404) has a substantially box-like conformation, or a substantially polyhedron or parallelepiped, possibly rectangle, or cylinder conformation, wherein said first half-shell (102, 202, 302, 402) comprises at least one upper face (102a, 202a, 302a, 402a), suitable for housing said first forming surface (108, 208, 308, 408) and a lateral wall (102b, 202b, 302b, 402b), which extends along the perimeter of said at least one upper face (102a, 202a, 302a, 402a), substantially perpendicular to the latter, and wherein said second half-shell (104, 204, 304, 404) comprises at least one lower face (104a, 204a, 304a, 404a), suitable for housing said second forming surface (110, 210, 310,410) and a side wall (104b, 204b, 304b, 404b), which extends along the perimeter of said at least one lower face (104a, 204a, 304a, 404a), substantially perpendicular to the latter.

10. Mold according to any one of the preceding claims, wherein said first forming surface (108, 208, 308, 408) comprises a base (108a, 208a, 308a, 408a), in recess with respect to the upper face (102a, 202a, 302a, 402a) or wherein said first forming surface (108, 208, 308, 408) comprises a base (108a, 208a, 308a, 408a) in recess with respect to said first perimeter edge (107, 207, 307, 407), and/or wherein said first forming surface (108, 208, 308, 408) comprises a lateral surface (108b, 208b, 308e, 408e) which extends along the perimeter of said base (108a, 208a, 308a, 408a) up to said first perimeter edge (107, 207, 307, 407) perpendicularly or inclined towards the outside with respect to said base (108a, 208a, 308a, 408a).

11. Mold according to the preceding claim, wherein said base (108a, 208a) comprises a series of ribs (115, 215) adapted to determine the formation of respective longitudinal grooves (1015, 2015) in the resulting spacer device, for example in a lower face (1008) of the tibial knee spacer device (1000) or in an upper face (2008) of the femoral knee spacer device (2000).

12. Mold according to any one of the preceding claims, wherein said first perimeter edge (107, 207, 307, 407) is connected to said upper face (102a, 202a, 302a, 402a) by means of a raised wall (108c, 208c, 308d, 408d) which is substantially inclined or perpendicular with respect to said upper face (102a, 202a, 302a, 402a), possibly away from said first forming surface (108, 208, 308, 408).

13. Mold according to any one of the preceding claims, wherein said second forming surface (110, 210, 310, 410) comprises a base (110a, 210a, 310a, 410a), which rises with respect to said lower face (104a, 204a, 304a, 404a), or wherein said second forming surface (110, 210, 310, 410) comprises a base (110a, 210a, 310a, 410a) at least partially in recess with respect to said second perimeter edge (109, 209, 309, 409), or wherein said second forming surface (110, 210, 310, 410) comprises a lateral surface (110b, 210b, 310e, 410e) which extends along the perimeter of said base (110a, 210a, 310a, 410a) up to said second perimeter edge (109, 209, 309, 409) perpendicularly or inclined towards the outside with respect to said base (110a, 210a, 310a, 410a).

14. Mold according to the preceding claim, wherein at said base (110a) there is a longitudinal central recess (116) corresponding to a protrusion with a substantially rectangular base (1016) present at an upper face (1010) of the tibial knee spacer device (1000) or wherein at said base (210a) there is a longitudinal central rib (216) corresponding to a recess with a substantially rectangular base (2016) present at a lower face (2010) of the femoral knee spacer device (2000).

15. Mold according to any one of the preceding claims, wherein said first forming surface (308, 408) comprises a stem portion (308a, 408a), a head portion (308b, 408b) and a connecting portion (308c, 408c) between said stem portion (308a, 408a) and said head portion (308b, 408b) and wherein said second forming surface (310, 410) comprises a stem portion (310a, 410a), a head portion (310b, 410b) and a connecting portion (310c, 410c) between said stem portion (310a , 410a) and said head portion (310b, 410b), so as to form said cavity (306, 406) between them.

16. Mold according to any one of the preceding claims, wherein at said side wall (102b, 202b, 302b, 402b) of said first half-shell (102, 202, 302, 402) and at said side wall (104b, 204b , 304b, 404b) of said second half-shell (104, 204, 304, 404) there is respectively a front in use area (102b1, 202b1, 302b1, 402b1 and 104b1, 204b1, 304b1, 404b1) and a rear in use area (102b2, 202b2, 302b2, 402b2 and 104b2, 204b2, 304b2, 404b2), wherein said rear in use area (102b2, 202b2, 302b2, 402b2 and 104b2, 204b2, 304b2, 404b2) is possibly opposite to said front in use area (102b1, 202b1, 302b1, 402b1 and 104b1, 204b1, 304b1, 404b1), wherein said mold comprises said handle (112, 212, 312, 412) at said front in use area (104b1, 204b1, 304b1, 404b1), or wherein said mold comprises said removable constraining structure (122, 222, 322, 422, 222') at said front in use area (104b1, 204b1, 304b1, 404b1).

17. Mold according to the previous claim, when dependent on claim 7, wherein said engagement seat (123b, 223b, 323b, 423b, 223b') is located at said front in use area (102b1, 202b1, 302b1, 402b1) of said side wall (102b, 202b, 302b, 402b) of said first half-shell (102, 202, 302, 402), wherein said trigger element (124, 224, 324, 4224, 224a') and/or said tooth or protrusion element (123a, 223a, 323a, 423a, 223a') is placed in use at said front in use area (104b1, 204b1, 304b1, 404b1) of said second half-shell (104, 204, 304, 404) and/or at said handle (112, 212, 312, 412) or vice-versa.

18. Mold according to any one of the preceding claims, wherein said removable constraining structure (122, 222, 322, 422, 222') and/or said coupling means (123, 223, 323, 423, 223'), comprise a bracket (123a1, 223a1, 323a1 , 423a1, 223a1') adapted to carry said tooth or protrusion element (123a, 223a, 323a, 423a, 223a'), wherein said bracket (123a1, 223a1, 323a1, 423a1, 223a1') has an elongated conformation which departs from said second half-shell (104, 204, 304, 404) or from said handle (112, 212, 312, 412) for a length such as to be able to bring said tooth or protrusion element (123a, 223a, 323a, 423a, 223a') at said engagement seat (123b, 223b, 323b, 423b, 223b'), when said second half- shell (104, 204, 304, 404) is brought into abutment against said first half-shell (102, 202, 302, 402) to close said mold (100, 200, 300 ,400), wherein said bracket (123a1, 223a1, 323a1, 423a1, 223a1') has a first end or area constrained to said second half-shell (104, 204, 304, 404) or to said handle (112, 212, 312, 412) and a second end or area, opposite the first end or area and further away from the latter with respect to said second half-shell (104, 204, 304, 404), and/or wherein said bracket (123a1, 223a1, 323a1, 423a1, 223a1') has a first face facing in use towards said first half-shell (102, 202, 302, 402) and a second face, opposite to the first face, wherein said tooth or protrusion element (123a, 223a, 323a, 423a, 223a') and/or said engagement seat (123b, 223b, 323b, 423b223b') is positioned at said second end or area and/or said first face and/or wherein said trigger element (124, 224, 324, 424, 224a') is positioned at said second face of said bracket (123a1, 223a1, 323a1,423a1, 223a1') or at said handle (112, 212, 312, 412).

19. Mold according to any one of the preceding claims, comprising hinge means (130, 230, 330, 430) positioned in said first half-shell (102, 202, 302, 402) and/or in said second half-shell (104, 204, 304 , 404), for example positioned at said rear in use area (102b2, 202b2, 302b2, 402b2) of said first half-shell (102, 202, 302, 402) and/or in said rear in use area (104b2, 204b2, 304b2, 404b2) of said second half-shell (104, 204, 304, 404), wherein said hinge means (130, 230, 330, 430) allow the reciprocal rotation of said second half-shell (104, 204, 304, 404) with respect to said first half-shell (102, 202, 302, 402).

20. Mold according to any one of the preceding claims, comprising extraction means (132, 232, 332, 432, 232') of the unidirectional or non-return type, adapted to extract the spacer device after its forming and to make said mold disposable (100, 200, 300, 400), wherein said extraction means (132, 232, 332, 432) comprise a button element (132a, 232a, 332a, 432a) equipped with a base (132b, 232b, 332b, 432b), suitable for being operated in use for the extraction of the formed spacer device, and a tip or end (132c, 232c, 332c, 432c) of a pressing body (132c1, 232c1, 332c1, 432c1), the latter having a substantially pin or peg conformation, wherein said tip or end (132c, 232c, 332c, 432c, 232c') faces into said cavity (106, 206, 306, 406) and/or into said additional cavity (106a) or wherein said extraction means (232') comprise a pressing body (32c1) comprising a base (32b) and a tip or end (32c), wherein said pressing body (32c1) has a thread, adapted to engage with a special seat (33) equipped with a nut screw and located at said first half-shell (2), further comprising a cap or lid (34) that can be housed or positioned at the tip (32c) and in a suitable seat or opening (34a) located at said first forming surface (108, 208, 308, 408), and/or a handle element (35) capable of being grasped by an operator for the purpose of screwing the extraction means (232') or comprising a discoidal base (36) adapted to comprise at least a pair of teeth (36a) made of a flexible material, adapted to make it possible to rotate the extraction means (232') but not to allow their unscrewing.

21. Mold according to any one of the preceding claims when depending on claim 2, wherein said channel (150) has a development corresponding to that of said first perimeter edge (107, 207, 307, 407) and/or of said second perimeter edge (109, 209, 309, 409) and of said first (108, 208, 308, 408) and/or of said second forming surface (110, 210, 310, 410), wherein said channel (150) has a surface, at least at the bottom, knurled or having a certain roughness or texture or finish and wherein said channel (150) has a recessed cavity with respect to the surface of said first half-shell or lower body (102, 202, 302, 402) and/or of said second half-shell or upper body (104, 204, 304, 404), with a wall substantially perpendicular or inclined with respect to the latter.

22. Method for forming a spacer device for the replacement of a joint prosthesis, such as a tibial knee spacer device, adapted to be implanted in use at the tibial bone of the knee joint, a femoral knee spacer device, adapted to be implanted in use at the femoral bone of the knee joint, a hip spacer device, adapted to be implanted in use at the femoral bone of the hip joint, or a shoulder spacer device, adapted to be implanted in use at a shoulder joint, comprising the following stages:
providing a mold (100, 200, 300, 400) according to any one of claims 1 to 21, comprising a first half-shell or lower body (102, 202, 302, 402) comprising a first forming surface (108, 208, 308, 408) delimited by a first perimeter edge (107, 207, 307, 407) and intended to mould at least a first portion of said spacer device, said first perimeter edge (107, 207, 307, 407) being continuous along all said first forming surface (108, 208, 308, 408), and
a second half-shell or upper body (104, 204, 304, 404) comprising a second forming surface (110, 210, 310, 410) delimited by a second perimeter edge (109, 209, 309, 409) and intended to mould at least a second portion of said spacer device, said second perimeter edge (109, 209, 309, 409) being continuous along all said second forming surface (110, 210, 310, 410),
providing a material which constitutes said spacer device and positioning and/or pouring, possibly in a manual way, said material in said first forming surface (108, 208, 308, 408) and/or in said second forming surface (110, 210, 310, 410),
coupling said first half-shell or lower body (102, 202, 302, 402) and said second half-shell or upper body (104, 204, 304, 404),
bring said first perimeter edge (107, 207, 307, 407) into abutment against said second perimeter edge (109, 209, 309, 409), so as to close said mold (100, 200, 300, 400) and delimit between these last, at said first forming surface (108, 208, 308, 408) and said second forming surface (110, 210, 310, 410), a cavity (106, 206, 306, 406) corresponding to the outer configuration of the spacer device,
waiting a time for the polymerization and/or hardening of said material and forming said spacer device,
wherein during said step of forming at least one of said first perimeter edge (107, 207, 307, 407) and said second perimeter edge (109, 209, 309, 409) cuts and/or eliminates any moulding burrs caused by the formation of said spacer device.

23. Method according to claim 22, wherein, during said step of forming said spacer device, some material of said spacer device flows or drips into a channel (150) adjacent to at least one of said first perimeter edge (107, 207, 307, 407) and said second perimeter edge ( 109, 209, 309, 409) and to at least one of said first forming surface (108, 208, 308, 408) and said second forming surface (110, 210, 310, 410) and adheres to and in said channel (150), so as to eliminate any moulding burrs caused by the formation of said spacer device.

24. Method according to claim 22, wherein said coupling step comprises operating a handle (112, 212, 312, 412), for example manually, and/or operating a removable constraining structure (122, 222, 322, 422, 222'), present in said second half-shell (104, 204, 304, 404) and/or in said first half-shell (102, 202, 302, 402), and removably constraining said first half-shell (102, 202, 302, 402) with said second half shell (104, 204, 304, 404).

25. Method according to claim 24, wherein said operating step comprises actuating coupling means (123, 223, 323, 423, 223'), for example snap-on, of the removable type of said removable constraining structure (122, 222, 322, 422, 222') and/or wherein said step of operating coupling means (123, 223, 323, 423, 223') comprises carrying a tooth or protrusion element (123a, 223a, 323a, 423a, 223a'), present at said second half-shell (104 , 204, 304, 404) at a suitable engagement seat (123b, 223b, 323b, 423b, 223b'), having a conformation corresponding or complementary to that of said tooth or protrusion element (123a, 223a, 323a, 423a, 223a'), made in said first half-shell (102, 202, 302, 402) or vice versa and engaging, for example by snapping, said tooth or protrusion element (123a, 223a, 323a,423a, 223a') in said engagement seat (123b, 223b, 323b, 423b, 223b').

26. Method according to any one of claims 22 to 25, comprising a step of extracting the spacer device formed by said mold (100, 200, 300, 400), wherein said step of extracting comprises moving away from each other said first half-shell (102, 202, 302, 402) and said second half-shell (104, 204, 304, 404), releasing said coupling means (123, 223, 323, 423, 223') making said tooth or protrusion element (123a, 223a, 323a, 423a, 223a') coming out from said engagement seat (123b, 223b, 323b, 423b, 223b'), and/or acting on a trigger element (124, 224, 324, 424, 224a') of said removable constraining structure (122, 222, 322 , 422, 222') and/or on said handle (112, 212, 312, 412) for said releasing and moving away step.

27. Method according to the preceding claim, wherein said extracting step comprises operating unidirectional or non-return type extraction means (132, 232, 332, 432, 232'), suitable for extracting the spacer device after its forming and making disposable said mold (100, 200, 300, 400), wherein said operating step comprises pressing a base (132b, 232b, 332b, 432b) of a button element (132a, 232a, 332a, 432a) so that a tip or end (132c, 232c, 332c, 432c) of a pressing body (132c1, 232c1, 332c1, 432c1) of said button element (132a, 232a, 332a, 432a), wherein said tip or end (132c, 232c, 332c, 432c) is faced into said cavity (106, 206, 306, 406) and/or into said additional cavity (106a), pushes said spacer device out of said cavity (106, 206, 306, 406) and/or of said additional cavity (106a) or wherein said operating step comprises screwing a pressing body (32c1) in a special seat (33) made in said first half-shell (102, 202, 302, 402) so that a tip or end (32c) of said pressing body (32c1) facing in said cavity (106, 206, 306, 406) and/or in said additional cavity (106a) pushes said spacer device out of said cavity (106, 206, 306, 406) and/or of said additional cavity (106a).

## Patentansprüche

1. Form (100, 200, 300, 400) zur Formung einer Abstandsvorrichtung zum Ersetzen einer Gelenkprothese, wie einer Tibia-Knie-Abstandsvorrichtung, die dazu geeignet ist, bei Verwendung am Tibiaknochen des Kniegelenks implantiert zu werden, einer Femur-Knie-Abstandsvorrichtung, die dazu geeignet ist, bei Verwendung am Femurknochen des Kniegelenks implantiert zu werden, einer Hüft-Abstandsvorrichtung, die dazu geeignet ist, bei Verwendung am Femurknochen des Hüftgelenks implantiert zu werden, oder einer Schulter-Abstandsvorrichtung, die dazu geeignet ist, bei Verwendung am Schultergelenk implantiert zu werden, umfassend:
eine erste Halbschale oder einen unteren Körper (102, 202, 302, 402), umfassend eine erste Formoberfläche (108, 208, 308, 408), die durch eine erste Umfangskante (107, 207, 307, 407) begrenzt ist und dazu bestimmt ist, mindestens einen ersten Abschnitt der besagten Abstandsvorrichtung zu formen, und
eine zweite Halbschale oder einen oberen Körper (104, 204, 304, 404), umfassend eine zweite Formoberfläche (110, 210, 310, 410), die durch eine zweite Umfangskante (109, 209, 309, 409) begrenzt ist und dazu bestimmt ist, mindestens einen zweiten Abschnitt der besagten Abstandsvorrichtung zu formen,
wobei die besagte erste Halbschale oder der besagte untere Körper (102, 202, 302, 402) und die besagte zweite Halbschale oder der besagte obere Körper (104, 204, 304, 404) in der Lage sind, bei Verwendung in einer lösbaren Weise verbunden zu werden, um für die besagte Form (100, 200, 300, 400) eine geschlossene Konfiguration zu definieren und zwischen sich, an der besagten ersten Formoberfläche (108, 208, 308, 408) und der besagten zweiten Formoberfläche (110, 210, 310, 410) sowie an der besagten ersten Umfangskante (107, 207, 307, 407) und der besagten zweiten Umfangskante (109, 209, 309, 409), einen Hohlraum (106, 206, 306, 406) zu begrenzen, der der äußeren Konfiguration der Abstandsvorrichtung entspricht,
wobei die erste Umfangskante (107, 207, 307, 407) in der Lage ist, bei Verwendung an der besagten zweiten Umfangskante (109, 209, 309, 409) anzuliegen und mit dieser in Kontakt zu kommen,
**dadurch gekennzeichnet, dass**
mindestens eine der besagten ersten Umfangskante (107, 207, 307, 407) und der besagten zweiten Umfangskante (109, 209, 309, 409) eine anliegende Schneidkante ist, um bei Verwendung irgendwelche Formgrate, die sich durch die Formung der besagten Abstandsvorrichtung ergeben, zu schneiden und/oder zu beseitigen, wenn sich die besagte Form in einer geschlossenen Konfiguration befindet, durch den Umstand, dass mindestens eine der besagten ersten Umfangskante (107, 207, 307, 407) und der besagten zweiten Umfangskante (109, 209, 309, 409) in Bezug auf die besagte erste Halbschale oder den besagten unteren Körper (102, 202, 302, 402) und die besagte zweite Halbschale oder den besagten oberen Körper (104, 204, 304, 404) erhöht ist, jeweils und durch den Umstand, dass die besagte erste Umfangskante (107, 207, 307, 407) entlang der gesamten ersten Formoberfläche (108, 208, 308, 408) durchgehend ist und die besagte zweite Umfangskante (109, 209, 309, 409) entlang der gesamten zweiten Formoberfläche (110, 210, 310, 410) durchgehend ist.

2. Form gemäß Anspruch 1, worin die besagte Form (100, 200, 300, 400) mindestens einen Kanal (150) umfasst, der an mindestens eine der besagten ersten Umfangskante (107, 207, 307, 407) und der besagten zweiten Umfangskante (109, 209, 309, 409) und an mindestens eine der besagten ersten Formoberfläche (108, 208, 308, 408) und der besagten zweiten Formoberfläche (110, 210, 310, 410) angrenzt, worin der besagte Kanal dazu ausgelegt ist, bei Verwendung überschüssiges Formmaterial für die besagte Abstandsvorrichtung zu erhalten.

3. Form nach Anspruch 1 oder 2, worin die besagte erste Umfangskante (107, 207, 307, 407) an die besagte zweite Umfangskante (109, 209, 309, 409) anliegt oder mit dieser gepaart ist oder dieser entspricht und/oder worin die besagte erste Umfangskante (107, 207, 307, 407) eine minimale Kontaktoberfläche mit der besagten zweiten Umfangskante (109, 209, 309, 409) aufweist und/oder worin die besagte erste Umfangskante (107, 207, 307, 407) und die besagte zweite Umfangskante (109, 209, 309, 409) flache anliegende Oberflächen aufweisen, die in direkten Kontakt miteinander kommen, worin die besagten anliegenden Oberflächen im Wesentlichen parallel zu einer Auflageebene für die besagte Form (100, 200, 300, 400) verlaufen oder beide dieselbe Neigung, zur Außenseite oder zur Innenseite hin, in Bezug auf den besagten Hohlraum (106, 206, 306, 406) aufweisen, oder worin die besagten Anliegeoberflächen beide geneigt sind, eine zur Außenseite hin und die andere zur Innenseite hin, in Bezug auf den besagten Hohlraum (106, 206, 306, 406), oder worin eine zwischen der besagten ersten Umfangskante (107, 207, 307, 407) und der besagten zweiten Umfangskante (109, 209, 309, 409) eine Anliegeoberfläche, zum Beispiel im Wesentlichen flach, aufweist, während die andere zwischen der besagten ersten Umfangskante (107, 207, 307, 407) und der besagten zweiten Umfangskante (109, 209, 309, 409) einen Anliegesitz aufweist, der als ein Annahme- oder Aufnahmebereich für die besagte Anliegeoberfläche geformt ist.

4. Form nach irgendeinem der vorangegangenen Ansprüche, umfassend eine lösbare Rückhaltestruktur (122, 222, 322, 422, 222'), die in der besagten zweiten Halbschale (104, 204, 304, 404) und/oder in der besagten ersten Halbschale (102, 202, 302, 402) vorhanden ist, die dazu ausgelegt ist, die besagte erste Halbschale (102, 202, 302, 402) lösbar an der besagten zweiten Halbschale (104, 204, 304, 404) festzuhalten, worin die besagte lösbare Rückhaltestruktur (122, 222, 322, 422, 222') mit Kopplungsmitteln (123, 223, 323, 423, 223'), zum Beispiel zum Einschnappen, ausgestattet ist, die zum Beispiel ein Zahn- oder Vorsprungselement (123a, 223a, 323a, 423a, 223a'), vorhanden an der besagten zweiten Halbschale (104, 204, 304, 404), umfassen, das dazu ausgelegt ist, in einer lösbaren Weise in einen geeigneten Eingriffssitz (123b, 223b, 323b, 423b, 223b') in Eingriff zu kommen, der eine Gestalt aufweist, die derjenigen des besagten Zahn- oder Vorsprungselements (123a, 223a, 323a, 423a, 223a'), ausgebildet in der besagten ersten Halbschale (102, 202, 302, 402), entspricht oder dazu komplementär ist oder umgekehrt.

5. Form nach irgendeinem der vorangegangenen Ansprüche, umfassend einen Griff (112, 212, 312, 412), der dazu ausgelegt ist, ergriffen zu werden, um die besagte zweite Halbschale oder den besagten oberen Körper (104, 204, 304, 404) gegen die besagte erste Halbschale oder den besagten unteren Körper (102, 202, 302, 402) in Anlage zu bringen und eine Kraft aufzubringen, um das Material, das die Abstandsvorrichtung bilden wird, zu komprimieren, worin der besagte Griff (112, 212, 312, 412) an der besagten zweiten Halbschale (104, 204, 304, 404) angeordnet ist und/oder lösbar ist.

6. Form nach den Ansprüchen 4 und 5, worin die besagte lösbare Rückhaltestruktur (122, 222, 322, 422, 222') ein Auslöseelement (124, 224, 324, 424, 224a') umfasst, worin das besagte Auslöseelement (124, 224, 324, 424, 224a') an dem besagten Griff (112, 212, 312, 412) angeordnet ist, wobei das besagte Auslöseelement (124, 224, 324, 424, 224a') in der Lage ist, während der Verwendung durch einen Bediener betätigt zu werden, sobald der Formungsschritt der besagten Abstandsvorrichtung abgeschlossen ist, und somit nach dem Abwarten einer vorbestimmten Zeit zum Aushärten des Materials, das die besagte Abstandsvorrichtung bildet, um die Freigabe und/oder Entkopplung des besagten Zahn- oder Vorsprungselements (123a, 223a, 323a, 423a, 223a') aus dem besagten Eingriffssitz (123b, 223b, 323b, 423b, 223b') zu veranlassen.

7. Form nach Anspruch 5 oder 6, worin der besagte Griff (112, 212, 312, 412) eine im Wesentlichen längliche und möglicherweise ergonomische Gestalt hat, mit einem im Wesentlichen rechteckigen Längsschnitt, mit abgerundeten Kanten und mit einem im Wesentlichen rechteckigen, quadratischen, kreisförmigen, dreieckigen Querschnitt usw., und/oder worin der besagte Griff (112, 212, 312, 412) im Inneren Verstärkungselemente (112a, 212a, 312a, 412a), zum Beispiel einige Rippen, umfasst.

8. Form nach irgendeinem der vorangegangenen Ansprüche, worin der besagte Hohlraum (106, 206) und die besagte erste Formoberfläche (108, 208) und/oder die besagte zweite Formoberfläche (110, 210) im Grundriss eine im Wesentlichen rechteckige, ovale oder C-förmige Gestalt aufweist, die derjenigen entspricht, die die resultierende Tibia-Abstandsvorrichtung oder Femur-Abstandsvorrichtung haben wird, und/oder worin die besagte Form (100) einen zusätzlichen Hohlraum (106a) umfasst, der bei Verwendung dazu ausgelegt ist, einen Schaft (1002) der besagten Tibia-Abstandsvorrichtung zu bilden, und an der besagten ersten Formoberfläche (108) angeordnet ist, worin der besagte zusätzliche Hohlraum (106a) und der besagte Hohlraum (106) aneinander angrenzen und in Fluidverbindung stehen.

9. Form nach irgendeinem der vorangegangenen Ansprüche, worin die besagte erste Halbschale (102, 202, 302, 402) und/oder die besagte zweite Halbschale (104, 204, 304, 404) eine im Wesentlichen kastenartige Gestalt oder eine im Wesentlichen polyedrische oder parallelepipedische, möglicherweise rechteckige, oder zylindrische Gestalt aufweist, worin die besagte erste Halbschale (102, 202, 302, 402) mindestens eine obere Fläche (102a, 202a, 302a, 402a), die zur Aufnahme der besagten ersten Formoberfläche (108, 208, 308, 408) geeignet ist, und eine Seitenwand (102b, 202b, 302b, 402b), die sich entlang des Umfangs der besagten mindestens einen oberen Fläche (102a, 202a, 302a, 402a) erstreckt, im Wesentlichen senkrecht zu letzterer, umfasst, und worin die besagte zweite Halbschale (104, 204, 304, 404) mindestens eine untere Fläche (104a, 204a, 304a, 404a), die zur Aufnahme der besagten zweiten Formoberfläche (110, 210, 310, 410) geeignet ist, und eine Seitenwand (104b, 204b, 304b, 404b), die sich entlang des Umfangs der besagten mindestens einen unteren Fläche (104a, 204a, 304a, 404a) erstreckt, im Wesentlichen senkrecht zu letzterer, umfasst.

10. Form nach irgendeinem der vorangegangenen Ansprüche, worin die besagte erste Formoberfläche (108, 208, 308, 408) eine Basis (108a, 208a, 308a, 408a) umfasst, die in Bezug auf die obere Fläche (102a, 202a, 302a, 402a) ausgespart ist, oder worin die besagte erste Formoberfläche (108, 208, 308, 408) eine Basis (108a, 208a, 308a, 408a) umfasst, die in Bezug auf die besagte erste Umfangskante (107, 207, 307, 407) ausgespart ist, und/oder worin die besagte erste Formoberfläche (108, 208, 308, 408) eine seitliche Oberfläche (108b, 208b, 308e, 408e) umfasst, die sich entlang des Umfangs der besagten Basis (108a, 208a, 308a, 408a) bis zur besagten ersten Umfangskante (107, 207, 307, 407) senkrecht oder zur Außenseite hin geneigt in Bezug auf die besagte Basis (108a, 208a, 308a, 408a) erstreckt.

11. Form nach dem vorangegangenen Anspruch, worin die besagte Basis (108a, 208a) eine Reihe von Rippen (115, 215) umfasst, die dazu ausgelegt sind, die Formung von jeweiligen Längsnuten (1015, 2015) in der resultierenden Abstandsvorrichtung zu veranlassen, zum Beispiel in einer unteren Fläche (1008) der Tibia-Knie-Abstandsvorrichtung (1000) oder in einer oberen Fläche (2008) der Femur-Knie-Abstandsvorrichtung (2000).

12. Form nach irgendeinem der vorangegangenen Ansprüche, worin die besagte erste Umfangskante (107, 207, 307, 407) mit der besagten oberen Fläche (102a, 202a, 302a, 402a) mittels einer erhöhten Wand (108c, 208c, 308d, 408d) verbunden ist, die im Wesentlichen geneigt oder senkrecht in Bezug auf die besagte obere Fläche (102a, 202a, 302a, 402a), möglicherweise von der besagten ersten Formoberfläche (108, 208, 308, 408) weg, verläuft.

13. Form nach irgendeinem der vorangegangenen Ansprüche, worin die besagte zweite Formoberfläche (110, 210, 310, 410) eine Basis (110a, 210a, 310a, 410a) umfasst, die sich in Bezug auf die besagte untere Fläche (104a, 204a, 304a, 404a) erhebt, oder worin die besagte zweite Formoberfläche (110, 210, 310, 410) eine Basis (110a, 210a, 310a, 410a) umfasst, die in Bezug auf die besagte zweite Umfangskante (109, 209, 309, 409) mindestens teilweise ausgespart ist, oder worin die besagte zweite Formoberfläche (110, 210, 310, 410) eine seitliche Oberfläche (110b, 210b, 310e, 410e) umfasst, die sich entlang des Umfangs der besagten Basis (110a, 210a, 310a, 410a) bis zur besagten zweiten Umfangskante (109, 209, 309, 409) senkrecht oder zur Außenseite hin geneigt in Bezug auf die besagte Basis (110a, 210a, 310a, 410a) erstreckt.

14. Form nach dem vorangegangenen Anspruch, worin an der besagten Basis (110a) eine längliche zentrale Aussparung (116) vorhanden ist, die einem Vorsprung mit einer im Wesentlichen rechteckigen Basis (1016) entspricht, der an einer oberen Fläche (1010) der Tibia-Knie-Abstandsvorrichtung (1000) vorhanden ist, oder worin an der besagten Basis (210a) eine längliche zentrale Rippe (216) vorhanden ist, die einer Aussparung mit einer im Wesentlichen rechteckigen Basis (2016) entspricht, die an einer unteren Fläche (2010) der Femur-Knie-Abstandsvorrichtung (2000) vorhanden ist.

15. Form nach irgendeinem der vorangegangenen Ansprüche, worin die besagte erste Formoberfläche (308, 408) einen Schaftabschnitt (308a, 408a), einen Kopfabschnitt (308b, 408b) und einen Verbindungsabschnitt (308c, 408c) zwischen dem besagten Schaftabschnitt (308a, 408a) und dem besagten Kopfabschnitt (308b, 408b) umfasst, und worin die besagte zweite Formoberfläche (310, 410) einen Schaftabschnitt (310a, 410a), einen Kopfabschnitt (310b, 410b) und einen Verbindungsabschnitt (310c, 410c) zwischen dem besagten Schaftabschnitt (310a, 410a) und dem besagten Kopfabschnitt (310b, 410b) umfasst, um zwischen diesen den besagten Hohlraum (306, 406) zu bilden.

16. Form nach irgendeinem der vorangegangenen Ansprüche, worin an der besagten Seitenwand (102b, 202b, 302b, 402b) der besagten ersten Halbschale (102, 202, 302, 402) und an der besagten Seitenwand (104b, 204b, 304b, 404b) der besagten zweiten Halbschale (104, 204, 304, 404) jeweils ein bei Verwendung vorderer Bereich (102b1, 202b1, 302b1, 402b1 und 104b1, 204b1, 304b1, 404b1) und ein bei Verwendung hinterer Bereich (102b2, 202b2, 302b2, 402b2 und 104b2, 204b2, 304b2, 404b2) vorhanden ist, worin der besagte bei Verwendung hintere Bereich (102b2, 202b2, 302b2, 402b2 und 104b2, 204b2, 304b2, 404b2) möglicherweise gegenüber dem besagten bei Verwendung vorderen Bereich (102b1, 202b1, 302b1, 402b1 und 104b1, 204b1, 304b1, 404b1) liegt, worin die besagte Form den besagten Griff (112, 212, 312, 412) an dem besagten bei Verwendung vorderen Bereich (104b1, 204b1, 304b1, 404b1) umfasst, oder worin die besagte Form die besagte lösbare Rückhaltestruktur (122, 222, 322, 422, 222') an dem besagten bei Verwendung vorderen Bereich (104b1, 204b1, 304b1, 404b1) umfasst.

17. Form nach dem vorangegangenen Anspruch, wenn abhängig von Anspruch 7, worin der besagte Eingriffssitz (123b, 223b, 323b, 423b, 223b') an dem besagten bei Verwendung vorderen Bereich (102b1, 202b1, 302b1, 402b1) der besagten Seitenwand (102b, 202b, 302b, 402b) der besagten ersten Halbschale (102, 202, 302, 402) angeordnet ist, worin das besagte Auslöseelement (124, 224, 324, 424, 224a') und/oder das besagte Zahn- oder Vorsprungselement (123a, 223a, 323a, 423a, 223a') bei Verwendung an dem besagten vorderen Bereich (104b1, 204b1, 304b1, 404b1) der besagten zweiten Halbschale (104, 204, 304, 404) und/oder an dem besagten Griff (112, 212, 312, 412) angeordnet ist oder umgekehrt.

18. Form nach irgendeinem der vorangegangenen Ansprüche, worin die besagte lösbare Rückhaltestruktur (122, 222, 322, 422, 222') und/oder die besagten Kopplungsmittel (123, 223, 323, 423, 223') eine Halterung (123a1, 223a1, 323a1, 423a1, 223a1') umfassen, die dazu ausgelegt ist, das besagte Zahn- oder Vorsprungselement (123a, 223a, 323a, 423a, 223a') zu tragen, worin die besagte Halterung (123a1, 223a1, 323a1, 423a1, 223a1') eine längliche Gestalt aufweist, die von der besagten zweiten Halbschale (104, 204, 304, 404) oder von dem besagten Griff (112, 212, 312, 412) über eine solche Länge ausgeht, dass sie in der Lage ist, das besagte Zahn- oder Vorsprungselement (123a, 223a, 323a, 423a, 223a') an den besagten Eingriffssitz (123b, 223b, 323b, 423b, 223b') zu bringen, wenn die besagte zweite Halbschale (104, 204, 304, 404) in Anlage gegen die besagte erste Halbschale(102, 202, 302, 402) gebracht wird, um die besagte Form (100, 200, 300 ,400) zu schließen, worin die besagte Halterung (123a1, 223a1, 323a1, 423a1, 223a1') ein erstes Ende oder einen ersten Bereich aufweist, das/der an der besagten zweiten Halbschale (104, 204, 304, 404) oder am besagten Griff (112, 212, 312, 412) festgehalten wird, und ein zweites Ende oder einen zweiten Bereich, das/der dem ersten Ende oder Bereich gegenüberliegt und von letzterem in Bezug auf die besagte zweite Halbschale (104, 204, 304, 404) weiter weg entfernt ist, und/oder worin die besagte Halterung (123a1, 223a1, 323a1, 423a1, 223a1') eine erste Fläche aufweist, die bei Verwendung der besagten ersten Halbschale (102, 202, 302, 402) zugewandt ist, und eine zweite Fläche, die der besagten ersten Fläche gegenüberliegt, worin das besagte Zahn- oder Vorsprungselement (123a, 223a, 323a, 423a, 223a') und/oder der besagte Eingriffssitz (123b, 223b, 323b, 423b, 223b') an dem besagten zweiten Ende oder Bereich und/oder der besagten ersten Fläche angeordnet ist und/oder worin das besagte Auslöseelement (124, 224, 324, 424, 224a') an der besagten zweiten Fläche der besagten Halterung (123a1, 223a1, 323a1, 423a1, 223a1') oder an dem besagten Griff (112, 212, 312, 412) angeordnet ist.

19. Form nach irgendeinem der vorangegangenen Ansprüche, umfassend Scharniermittel (130, 230, 330, 430), die in der besagten ersten Halbschale (102, 202, 302, 402) und/oder in der besagten zweiten Halbschale (104, 204, 304, 404), zum Beispiel an dem besagten bei Verwendung hinteren Bereich (102b2, 202b2, 302b2, 402b2) der besagten ersten Halbschale (102, 202, 302, 402) und/oder in dem besagten bei Verwendung hinteren Bereich (104b2, 204b2, 304b2, 404b2) der besagten zweiten Halbschale (104, 204, 304, 404) angeordnet sind, worin die besagten Scharniermittel (130, 230, 330, 430) die wechselseitige Drehung der besagten zweiten Halbschale (104, 204, 304, 404) in Bezug auf die besagte erste Halbschale (102, 202, 302, 402) ermöglichen.

20. Form nach irgendeinem der vorangegangenen Ansprüche, umfassend Ausziehmittel (132, 232, 332, 432, 232') des unidirektionalen und/oder nicht-rückkehrenden Typs, die dazu ausgelegt sind, die Abstandsvorrichtung nach ihrer Formung herauszuziehen und die besagte Form (100, 200, 300, 400) entsorgbar zu machen, worin die besagten Ausziehmittel (132, 232, 332, 432) ein Knopfelement (132a, 232a, 332a, 432a), ausgestattet mit einer Basis (132b, 232b, 332b, 432b), umfassen, das geeignet ist, bei Verwendung für das Ausziehen der geformten Abstandsvorrichtung angewendet zu werden, und eine Spitze oder ein Ende (132c, 232c, 332c, 432c) eines Druckkörpers (132c1, 232c1, 332c1, 432c1), wobei letzterer im Wesentlichen die Gestalt eines Stifts oder Zapfens aufweist, worin die besagte Spitze oder das besagte Ende (132c, 232c, 332c, 432c, 232c') in den besagten Hohlraum (106, 206, 306, 406) und/oder in den besagten zusätzlichen Hohlraum (106a) gerichtet ist, oder worin die besagten Ausziehmittel (232') einen Druckkörper (32c1) umfassend eine Basis (32b) und eine Spitze oder ein Ende (32c) umfassen, worin der besagte Druckkörper (32c1) ein Gewinde aufweist, das dazu ausgelegt ist, in einen speziellen Sitz (33) einzugreifen, der mit einer Mutterschraube ausgestattet ist und an der besagten ersten Halbschale (2) angeordnet ist, ferner umfassend eine Kappe oder einen Deckel (34), der an der Spitze (32c) und in einem geeigneten Sitz oder in einer geeigneten Öffnung (34a) aufgenommen und/oder angeordnet werden kann, der/die an der besagten ersten Formoberfläche (108, 208, 308, 408) angeordnet ist, und/oder ein Griffelement (35), das dazu fähig ist, von einem Bediener für den Zweck des Einschraubens der Ausziehmittel (232') ergriffen zu werden oder umfassend eine scheibenförmige Basis (36), die dazu ausgelegt ist, mindestens ein Paar von Zähnen (36a) aus einem flexiblen Material zu umfassen, die dazu ausgelegt sind, das Drehen der Ausziehmittel (232') zu ermöglichen, ihr Herausdrehen jedoch nicht erlauben.

21. Form nach irgendeinem der vorangegangenen Ansprüche, wenn abhängig von Anspruch 2, worin der besagte Kanal (150) einen Verlauf aufweist, der demjenigen der besagten ersten Umfangskante (107, 207, 307, 407) und/oder der besagten zweiten Umfangskante (109, 209, 309, 409) und der besagten ersten (108, 208, 308, 408) und/oder der besagten zweiten Formoberfläche (110, 210, 310, 410) entspricht, worin der besagte Kanal (150) eine Oberfläche, mindestens am Boden, aufweist, die gerändelt ist oder eine gewisse Rauheit oder Textur oder Oberflächenbeschaffenheit hat, und worin der besagte Kanal (150) einen ausgesparten Hohlraum in Bezug auf die Oberfläche der besagten ersten Halbschale oder des besagten unteren Körpers (102, 202, 302, 402) und/oder der besagten zweiten Halbschale oder des besagten oberen Körpers (104, 204, 304, 404) aufweist, mit einer Wand, die in Bezug auf letztere(n) im Wesentlichen senkrecht oder geneigt ist.

22. Verfahren zur Formung einer Abstandsvorrichtung zur Ersetzung einer Gelenkprothese, wie einer Tibia-Knie-Abstandsvorrichtung, die dazu ausgelegt ist, bei Verwendung am Tibiaknochen des Kniegelenks implantiert zu werden, einer Femur-Knie-Abstandsvorrichtung, die dazu ausgelegt ist, bei Verwendung am Femurknochen des Kniegelenks implantiert zu werden, einer Hüft-Abstandsvorrichtung, die dazu ausgelegt ist, bei Verwendung am Femurknochen des Hüftgelenks implantiert zu werden, oder einer Schulter-Abstandsvorrichtung, die dazu ausgelegt ist, bei Verwendung an einem Schultergelenk implantiert zu werden, umfassend die folgenden Schritte:
Bereitstellen einer Form (100, 200, 300, 400) nach irgendeinem der Ansprüche 1 bis 21, umfassend eine erste Halbschale oder einen unteren Körper (102, 202, 302, 402), umfassend eine erste Formoberfläche (108, 208, 308, 408), die durch eine erste Umfangskante (107, 207, 307, 407) begrenzt ist und dazu bestimmt ist, mindestens einen ersten Abschnitt der besagten Abstandsvorrichtung zu formen, wobei die besagte erste Umfangskante (107, 207, 307, 407) durchgehend entlang der gesamten besagten ersten Formoberfläche (108, 208, 308, 408) verläuft, und
eine zweite Halbschale oder einen oberen Körper (104, 204, 304, 404), umfassend eine zweite Formoberfläche (110, 210, 310, 410), die durch eine zweite Umfangskante (109, 209, 309, 409) begrenzt ist und dazu bestimmt ist, mindestens einen zweiten Abschnitt der besagten Abstandsvorrichtung zu formen, wobei die besagte zweite Umfangskante (109, 209, 309, 409) durchgehend entlang der gesamten besagten zweiten Formoberfläche (110, 210, 310, 410) verläuft,
Bereitstellen eines Materials, das die besagte Abstandsvorrichtung bildet, und Anordnen und/oder Gießen, möglicherweise auf manuelle Weise, des besagten Materials in die besagte erste Formoberfläche (108, 208, 308, 408) und/oder in die besagte zweite Formoberfläche (110, 210, 310, 410),
Koppeln der besagten ersten Halbschale oder des besagten unteren Körpers (102, 202, 302, 402) und der besagten zweiten Halbschale oder des besagten oberen Körpers (104, 204, 304, 404),
Bringen der besagten ersten Umfangskante (107, 207, 307, 407) in Anlage gegen die besagte zweite Umfangskante (109, 209, 309, 409), um die besagte Form (100, 200, 300, 400) zu schließen und zwischen diesen letzteren, an der besagten ersten Formoberfläche (108, 208, 308, 408) und der besagten zweiten Formoberfläche (110, 210, 310, 410), einen Hohlraum (106, 206, 306, 406) zu begrenzen, der der äußeren Konfiguration der Abstandsvorrichtung entspricht,
Abwarten einer Zeit für die Polymerisation und/oder Aushärtung des besagten Materials und Formung der besagten Abstandsvorrichtung,
worin während des besagten Schritts der Formung mindestens eine der besagten ersten Umfangskante (107, 207, 307, 407) und der besagten zweiten Umfangskante (109, 209, 309, 409) jegliche Formgrate, die durch die Formung der besagten Abstandsvorrichtung verursacht werden, schneidet und/oder beseitigt.

23. Verfahren nach Anspruch 22, worin, während des besagten Schritts der Formung der besagten Abstandsvorrichtung, etwas Material der besagten Abstandsvorrichtung in einen Kanal (150) fließt oder tropft, der an mindestens eine der besagten ersten Umfangskante (107, 207, 307, 407) und der besagten zweiten Umfangskante (109, 209, 309, 409) und an mindestens eine der besagten ersten Formoberfläche (108, 208, 308, 408) und der besagten zweiten Formoberfläche (110, 210, 310, 410) angrenzt und in dem besagten Kanal (150) anhaftet, um jegliche Formgrate, die durch die Formung der besagten Abstandsvorrichtung verursacht werden, zu beseitigen.

24. Verfahren nach Anspruch 22, worin der besagte Kopplungsschritt das Betätigen eines Griffs (112, 212, 312, 412), zum Beispiel manuell, und/oder das Betätigen einer lösbaren Rückhaltestruktur (122, 222, 322, 422, 222'), die in der besagten zweiten Halbschale (104, 204, 304, 404) und/oder in der besagten ersten Halbschale (102, 202, 302, 402) vorhanden ist und die besagte erste Halbschale (102, 202, 302, 402) lösbar an der besagten zweiten Halbschale (104, 204, 304, 404) festhält, umfasst.

25. Verfahren nach Anspruch 24, worin der besagte Arbeitsschritt das Betätigen von Kopplungsmitteln (123, 223, 323, 423, 223'), zum Beispiel zum Einschnappen, des lösbaren Typs der besagten lösbaren Rückhaltestruktur (122, 222, 322, 422, 222') umfasst und/oder worin der besagte Schritt des Betätigens der Kopplungsmittel (123, 223, 323, 423, 223') das Tragen eines Zahn- oder Vorsprungselements (123a, 223a, 323a, 423a, 223a'), vorhanden an der besagten zweiten Halbschale (104, 204, 304, 404) an einem geeigneten Eingriffssitz (123b, 223b, 323b, 423b, 223b'), mit einer Gestalt, die derjenigen des besagten Zahn- oder Vorsprungselements (123a, 223a, 323a, 423a, 223a'), das in der besagten ersten Halbschale (102, 202, 302, 402) ausgebildet ist, entspricht oder komplementär dazu ist oder umgekehrt und des Eingreifens, zum Beispiel durch Einschnappen, des besagten Zahn- oder Vorsprungselements (123a, 223a, 323a,423a, 223a') in den besagten Eingriffssitz (123b, 223b, 323b, 423b, 223b') umfasst.

26. Verfahren nach irgendeinem der Ansprüche 22 bis 25, umfassend einen Schritt des Ausziehens der durch die besagte Form (100, 200, 300, 400) geformten Abstandsvorrichtung, worin der besagte Schritt des Ausziehens das Wegbewegen der besagten ersten Halbschale (102, 202, 302, 402) und der besagten zweiten Halbschale (104, 204, 304, 404) voneinander, das Freigeben der besagten Kopplungsmittel (123, 223, 323, 423, 223') durch Austretenlassen des besagten Zahn- oder Vorsprungselements (123a, 223a, 323a, 423a, 223a') aus dem besagten Eingriffssitz (123b, 223b, 323b, 423b, 223b') und/oder das Einwirken auf ein Auslöseelement (124, 224, 324, 424, 224a') der besagten lösbaren Rückhaltestruktur (122, 222, 322, 422, 222') und/oder auf den besagten Griff (112, 212, 312, 412) für den besagten Schritt des Lösens und Wegbewegens umfasst.

27. Verfahren nach dem vorangegangenen Anspruch, worin der besagte Ausziehschritt das Betätigen von Ausziehmitteln (132, 232, 332, 432, 232') des unidirektionalen oder nicht-rückkehrenden Typs umfasst, der zum Ausziehen der Abstandsvorrichtung nach ihrem Formen und Entsorgbar-Machen der besagten Form (100, 200, 300, 400) geeignet sind, worin der besagte Arbeitsschritt das Drücken einer Basis (132b, 232b, 332b, 432b) eines Knopfelements (132a, 232a, 332a, 432a) umfasst, so dass eine Spitze oder ein Ende (132c, 232c, 332c, 432c) eines Druckkörpers (132c1, 232c1, 332c1, 432c1) des besagten Knopfelements (132a, 232a, 332a, 432a), worin die besagte Spitze oder das besagte Ende (132c, 232c, 332c, 432c) in den besagten Hohlraum (106, 206, 306, 406) und/oder in den besagten zusätzlichen Hohlraum (106a) gerichtet ist, die besagte Abstandsvorrichtung aus dem besagten Hohlraum (106, 206, 306, 406) und/oder aus dem besagten zusätzlichen Hohlraum (106a) herausdrückt, oder worin der besagte Arbeitsschritt das Einschrauben eines Druckkörpers (32c1) in einen speziellen Sitz (33) umfasst, der in der besagten ersten Halbschale (102, 202, 302, 402) ausgebildet ist, so dass eine Spitze oder ein Ende (32c) des besagten Druckkörpers (32c1), die/das in den besagten Hohlraum (106, 206, 306, 406) und/oder in den besagten zusätzlichen Hohlraum (106a) gerichtet ist, die besagte Abstandsvorrichtung aus dem besagten Hohlraum (106, 206, 306, 406) und/oder aus dem besagten zusätzlichen Hohlraum (106a) herausdrückt.

## Revendications

1. Moule (100, 200, 300, 400) pour former un dispositif d'espacement pour remplacer une prothèse articulaire, tel qu'un dispositif d'espacement tibial du genou, apte à être implanté pendant l'utilisation au niveau de l'os tibial de l'articulation du genou, un dispositif d'espacement fémoral du genou, apte à être implanté pendant l'utilisation au niveau de l'os fémoral de l'articulation du genou, un dispositif d'espacement de la hanche, apte à être implanté pendant l'utilisation au niveau de l'os fémoral de l'articulation de la hanche, ou un dispositif d'espacement de l'épaule, apte à être implanté pendant l'utilisation au niveau de l'articulation de l'épaule, comprenant :
une première demi-coque ou corps inférieur (102, 202, 302, 402) comprenant une première surface de formage (108, 208, 308, 408) délimitée par un premier bord périmétrique (107, 207, 307, 407) et destinée à mouler au moins une première portion dudit dispositif d'espacement, et
une deuxième demi-coque ou corps supérieur (104, 204, 304, 404) comprenant une deuxième surface de formage (110, 210, 310, 410) délimitée par un deuxième bord périmétrique (109, 209, 309, 409) et destinée à mouler au moins une deuxième portion dudit dispositif d'espacement,
ladite première demi-coque ou corps inférieur (102, 202, 302, 402) et ladite deuxième demi-coque ou corps supérieur (104, 204, 304, 404) pouvant être couplées, pendant l'utilisation, de manière amovible, afin de définir pour ledit moule (100, 200, 300, 400) une configuration fermée et de délimiter entre elles, au niveau de ladite première surface de formage (108, 208, 308, 408) et de ladite deuxième surface de formage (110, 210, 310, 410), ainsi qu'au niveau dudit premier bord périmétrique (107, 207, 307, 407) et dudit deuxième bord périmétrique (109, 209, 309, 409), une cavité (106, 206, 306, 406) correspondant à la configuration externe du dispositif d'espacement,
ledit premier bord périmètrique (107, 207, 307, 407) pouvant venir en butée et entrer en contact, pendant l'utilisation, avec ledit deuxième bord périmétrique (109, 209, 309, 409),
**caractérisé en ce que**
au moins l'un dudit premier bord périmétrique (107, 207, 307, 407) et dudit deuxième bord périmétrique (109, 209, 309, 409) est un bord de coupe en butée, afin de couper et/ou d'éliminer pendant l'utilisation les bavures de moulage déterminées par le formage dudit dispositif d'espacement lorsque ledit moule est en configuration fermée, **en ce que** au moins un dudit premier bord périmétrique (107, 207, 307, 407) et dudit deuxième bord périmétrique(109, 209, 309, 409) est relevé par rapport à ladite première demi-coque ou au corps inférieur (102, 202, 302, 402) et à ladite deuxième demi-coque ou au corps supérieur (104, 204, 304, 404), respectivement et **en ce que** ledit premier bord périmétrique (107, 207, 307, 407) est continu sur toute ladite première surface de formage (108, 208, 308, 408) et ledit deuxième bord périmétrique (109, 209, 309, 409) est continu sur toute ladite deuxième surface de formage (110, 210, 310, 410).

2. Moule selon la revendication 1, dans lequel ledit moule (100, 200, 300, 400) comprend au moins un canal (150) adjacent à au moins un dudit premier bord périmétrique (107, 207, 307, 407) et dudit deuxième bord périmétrique (109, 209, 309, 409) et à au moins une de ladite première surface de formage (108, 208, 308, 408) et de ladite deuxième surface de formage (110, 210, 310, 410), dans lequel ledit canal est adapté pour recevoir pendant l'utilisation du matériau de formage en excès pour ledit dispositif d'espacement.

3. Moule selon la revendication 1 ou 2, dans lequel ledit premier bord périmétrique (107, 207, 307, 407) est en butée contre ou s'enclenche avec ou correspond audit deuxième bord périmétrique (109, 209, 309, 409) et/ou dans lequel ledit premier bord périmétrique (107, 207, 307, 407) présente une surface de contact minimale avec ledit deuxième bord périmétrique (109, 209, 309, 409) et/ou dans lequel ledit premier bord périmétrique (107, 207, 307, 407) et ledit deuxième bord périmétrique (109, 209, 309, 409) présentent des surfaces planes en butée qui entrent en contact direct l'une avec l'autre, dans lequel lesdites surfaces en butée sont sensiblement parallèles à un plan de soutien dudit moule (100, 200, 300, 400) ou présentant toutes deux la même inclinaison, vers l'extérieur ou vers l'intérieur, par rapport à ladite cavité (106, 206, 306, 406), ou dans lequel lesdites surfaces de butée sont toutes deux inclinées, l'une vers l'extérieur et l'autre vers l'intérieur, par rapport à ladite cavité (106, 206, 306, 406), ou dans lequel un entre ledit premier bord périmétrique (107, 207, 307, 407) et ledit deuxième bord périmétrique (109, 209, 309, 409) présente une surface de butée, par exemple sensiblement plate, tandis que l'autre entre ledit premier bord périmétrique (107, 207, 307, 407) et ledit deuxième bord périmétrique (109, 209, 309, 409) présente un siège de butée façonné comme une zone d'acceptation ou de logement pour ladite surface de butée.

4. Moule selon l'une quelconque des revendications précédentes, comprenant une structure de contrainte amovible (122, 222, 322, 422, 222'), existant dans ladite deuxième demi-coque (104, 204, 304, 404) et/ou dans ladite première demi-coque (102, 202, 302, 402), adaptée pour contraindre de manière amovible ladite première demi-coque (102, 202, 302, 402) avec ladite deuxième demi-coque (104, 204, 304, 404), dans lequel ladite structure de contrainte amovible (122, 222, 322, 422, 222') est dotée de moyens de couplage (123, 223, 323, 423, 223'), par exemple à encliquetage, qui comprend par exemple un élément de dent ou de protubérance (123a, 223a, 323a, 423a, 223a'), existant au niveau de ladite deuxième demi-coque (104, 204, 304, 404) adapté pour s'engager de manière amovible dans un siège d'engagement approprié (123b, 223b, 323b, 423b, 223b'), présentant une conformation correspondant ou complémentaire à celle dudit élément de dent ou de protubérance (123a, 223a, 323a, 423a, 223a'), fabriqué dans ladite première demi-coque (102, 202, 302, 402) ou vice versa.

5. Moule selon l'une quelconque des revendications précédentes, comprenant une poignée (112, 212, 312, 412) adaptée pour être saisie pour amener ladite deuxième demi-coque ou corps supérieur (104, 204, 304, 404) en butée contre ladite première demi-coque ou corps inférieur (102, 202, 302, 402) et pour appliquer une force pour comprimer le matériau qui constituera le dispositif d'espacement, dans lequel ladite poignée (112, 212, 312, 412) est placée au niveau de ladite deuxième demi-coque (104, 204, 304, 404) et/ou est amovible.

6. Moule selon la revendication 4 et 5, dans lequel ladite structure de contrainte amovible (122, 222, 322, 422, 222') comprend un élément de déclenchement (124, 224, 324, 424, 224a'), ledit élément de déclenchement (124, 224, 324, 424, 224a') étant placé au niveau de ladite poignée (112, 212, 312, 412), ledit élément de déclenchement (124, 224, 324, 424, 224a') pouvant être actionné pendant l'utilisation par un opérateur, une fois l'étape de formage dudit dispositif d'espacement terminée, et donc après avoir attendu un temps prédéterminé pour le durcissement du matériau constituant ledit dispositif d'espacement, pour déterminer le dégagement et/ou le découplage dudit élément de dent ou de protubérance (123a, 223a, 323a, 423a, 223a') hors dudit siège d'engagement (123b, 223b, 323b, 423b, 223b').

7. Moule selon la revendication 5 ou 6, dans lequel ladite poignée (112, 212, 312, 412) présente une conformation sensiblement allongée et éventuellement ergonomique, avec une section longitudinale sensiblement rectangulaire, avec des bords arrondis et avec une section transversale sensiblement rectangulaire, carrée, circulaire, triangulaire, etc., et/ou dans lequel ladite poignée (112, 212, 312, 412) comprend intérieurement des éléments de renforcement (112a, 212a, 312a, 412a), par exemple des nervures.

8. Moule selon l'une quelconque des revendications précédentes, dans lequel ladite cavité (106, 206) et ladite première surface de formage (108, 208) et/ou ladite deuxième surface de formage (110, 210) présente une conformation plane sensiblement rectangulaire, ovale ou en forme de C, correspondant à celle que le dispositif d'espacement tibial ou fémoral résultant aura, et/ou dans lequel ledit moule (100) comprend une cavité supplémentaire (106a), adaptée pendant l'utilisation pour former une tige (1002) dudit dispositif d'espacement tibial, et placée au niveau de ladite première surface de formage (108), dans lequel ladite cavité supplémentaire (106a) et ladite cavité (106) sont contiguës l'une à l'autre et en connexion fluide.

9. Moule selon l'une quelconque des revendications précédentes, dans lequel ladite première demi-coque (102, 202, 302, 402) et/ou ladite deuxième demi-coque (104, 204, 304, 404) présente une conformation sensiblement en forme de boîte, ou une conformation sensiblement de polyèdre ou de parallélépipède, éventuellement de rectangle, ou de cylindre, dans lequel ladite première demi-coque (102, 202, 302, 402) comprend au moins une face supérieure (102a, 202a, 302a, 402a), apte à loger ladite première surface de formage (108, 208, 308, 408) et une paroi latérale (102b, 202b, 302b, 402b), qui s'étend le long du périmètre de ladite au moins une face supérieure (102a, 202a, 302a, 402a), sensiblement perpendiculaire à cette dernière, et dans lequel ladite deuxième demi-coque (104, 204, 304, 404) comprend au moins une face inférieure (104a, 204a, 304a, 404a), apte à loger ladite deuxième surface de formage (110, 210, 310, 410) et une paroi latérale (104b, 204b, 304b, 404b), qui s'étend le long du périmètre de ladite au moins une face inférieure (104a, 204a, 304a, 404a), sensiblement perpendiculaire à cette dernière.

10. Moule selon l'une quelconque des revendications précédentes, dans lequel ladite première surface de formage (108, 208, 308, 408) comprend une base (108a, 208a, 308a, 408a) en renfoncement par rapport à la face supérieure (102a, 202a, 302a, 402a), ou dans lequel ladite première surface de formage (108, 208, 308, 408) comprend une base (108a, 208a, 308a, 408a) en renfoncement par rapport audit premier bord périmétrique (107, 207, 307, 407), et/ou dans lequel ladite première surface de formage (108, 208, 308, 408) comprend une surface latérale (108b, 208b, 308e, 408e) qui s'étend le long du périmètre de ladite base (108a, 208a, 308a, 408a) jusqu'audit premier bord périmétrique (107, 207, 307, 407) perpendiculairement ou inclinée vers l'extérieur par rapport à ladite base (108a, 208a, 308a, 408a).

11. Moule selon la revendication précédente, dans lequel ladite base (108a, 208a) comprend une série de nervures (115, 215) adaptées pour déterminer la formation de rainures longitudinales respectives (1015, 2015) dans le dispositif d'espacement résultant, par exemple dans une face inférieure (1008) du dispositif d'espacement tibial du genou (1000) ou dans une face supérieure (2008) du dispositif d'espacement fémoral du genou (2000).

12. Moule selon l'une quelconque des revendications précédentes, dans lequel ledit premier bord périmétrique (107, 207, 307, 407) est connecté à ladite face supérieure (102a, 202a, 302a, 402a) au moyen d'une paroi relevée (108c, 208c, 308d, 408d) qui est sensiblement inclinée ou perpendiculaire par rapport à ladite face supérieure (102a, 202a, 302a, 402a), éventuellement éloignée de ladite première surface de formage (108, 208, 308, 408).

13. Moule selon l'une quelconque des revendications précédentes, dans lequel ladite deuxième surface de formage (110, 210, 310, 410) comprend une base (110a, 210a, 310a, 410a) qui s'élève par rapport à ladite face inférieure (104a, 204a, 304a, 404a), ou dans lequel ladite deuxième surface de formage (110, 210, 310, 410) comprend une base (110a, 210a, 310a, 410a) au moins partiellement en renfoncement par rapport audit deuxième bord périmétrique (109, 209, 309, 409), ou dans lequel ladite deuxième surface de formage (110, 210, 310, 410) comprend une surface latérale (110b, 210b, 310e, 410e) qui s'étend le long du périmètre de ladite base (110a, 210a, 310a, 410a) jusqu'audit deuxième bord périmétrique (109, 209, 309, 409) perpendiculairement ou incliné vers l'extérieur par rapport à ladite base (110a, 210a, 310a, 410a).

14. Moule selon la revendication précédente, dans lequel au niveau de ladite base (110a) il existe un renfoncement central longitudinal (116) correspondant à une protubérance avec une base sensiblement rectangulaire (1016) existant au niveau d'une face supérieure (1010) du dispositif d'espacement tibial du genou (1000) ou dans lequel au niveau de ladite base (210a) il existe une nervure centrale longitudinale (216) correspondant à un renfoncement avec une base sensiblement rectangulaire (2016) existant sur une face inférieure (2010) du dispositif d'espacement fémoral du genou (2000).

15. Moule selon l'une quelconque des revendications précédentes, dans lequel ladite première surface de formage (308, 408) comprend une portion de tige (308a, 408a), une portion de tête (308b, 408b) et une portion de connexion (308c, 408c) entre ladite portion de tige (308a, 408a) et ladite portion de tête (308b, 408b), et dans lequel ladite deuxième surface de formage (310, 410) comprend une portion de tige (310a, 410a), une portion de tête (310b, 410b) et une portion de connexion (310c, 410c) entre ladite portion de tige (310a, 410a) et ladite portion de tête (310b, 410b), afin de former ladite cavité (306, 406) entre elles.

16. Moule selon l'une quelconque des revendications précédentes, dans lequel, au niveau de ladite paroi latérale (102b, 202b, 302b, 402b) de ladite première demi-coque (102, 202, 302, 402) et au niveau de ladite paroi latérale (104b, 204b, 304b, 404b) de ladite deuxième demi-coque (104, 204, 304, 404), il existe respectivement une zone avant pendant l'utilisation (102b1, 202b1, 302b1, 402b1 et 104b1, 204b1, 304b1, 404b1) et une zone arrière pendant l'utilisation (102b2, 202b2, 302b2, 402b2 et 104b2, 204b2, 304b2, 404b2), dans lequel ladite zone arrière pendant l'utilisation (102b2, 202b2, 302b2, 402b2 et 104b2, 204b2, 304b2, 404b2) est éventuellement opposée à ladite zone avant pendant l'utilisation (102b1, 202b1, 302b1, 402b1 et 104b1, 204b1, 304b1, 404b1), dans lequel ledit moule comprend ladite poignée (112, 212, 312, 412) au niveau de ladite zone avant pendant l'utilisation (104b1, 204b1, 304b1, 404b1), ou dans lequel ledit moule comprend ladite structure de contrainte amovible (122, 222, 322, 422, 222') au niveau de ladite zone avant pendant l'utilisation (104b1, 204b1, 304b1, 404b1).

17. Moule selon la revendication précédente, lorsqu'il dépend de la revendication 7, dans lequel ledit siège d'engagement (123b, 223b, 323b, 423b, 223b') est situé au niveau de ladite zone avant pendant l'utilisation (102b1, 202b1, 302b1, 402b1) de ladite paroi latérale (102b, 202b, 302b, 402b) de ladite première demi-coque (102, 202, 302, 402), dans lequel ledit élément de déclenchement (124, 224, 324, 424, 224a') et/ou ledit élément de dent ou de protubérance (123a, 223a, 323a, 423a, 223a') est placé pendant l'utilisation au niveau de ladite zone avant pendant l'utilisation (104b1, 204b1, 304b1, 404b1) de ladite deuxième demi-coque (104, 204, 304, 404) et/ou au niveau de ladite poignée (112, 212, 312, 412) ou vice-versa.

18. Moule selon l'une quelconque des revendications précédentes, dans lequel ladite structure de contrainte amovible (122, 222, 322, 422, 222') et/ou lesdits moyens de couplage (123, 223, 323, 423, 223') comprennent un support (123a1, 223a1, 323a1, 423a1, 223a1') adapté pour porter ledit élément de dent ou de protubérance (123a, 223a, 323a, 423a, 223a'), dans lequel ledit support (123a1, 223a1, 323a1, 423a1, 223a1') présente une conformation allongée qui s'écarte de ladite deuxième demi-coque (104, 204, 304, 404) ou de ladite poignée (112, 212, 312, 412) sur une longueur permettant d'amener ledit élément de dent ou de protubérance (123a, 223a, 323a, 423a, 223a') au niveau dudit siège d'engagement (123b, 223b, 323b, 423b, 223b'), lorsque ladite deuxième demi-coque (104, 204, 304, 404) est mise en butée contre ladite première demi-coque (102, 202, 302, 402) pour fermer ledit moule (100, 200, 300, 400), dans lequel ledit support (123a1, 223a1, 323a1, 423a1, 223a1') présente une première extrémité ou zone contrainte à ladite deuxième demi-coque (104, 204, 304, 404) ou à ladite poignée (112, 212, 312, 412) et une deuxième extrémité ou zone, opposée à la première extrémité ou zone et plus éloignée de cette dernière par rapport à ladite deuxième demi-coque (104, 204, 304, 404), et/ou dans lequel ledit support (123a1, 223a1, 323a1, 423a1, 223a1') présente une première face orientée pendant l'utilisation vers ladite première demi-coque (102, 202, 302, 402) et une deuxième face, opposée à la première face, dans laquelle ledit élément de dent ou de protubérance (123a, 223a, 323a, 423a, 223a') et/ou ledit siège d'engagement (123b, 223b, 323b, 423b, 223b') est positionné au niveau de ladite deuxième extrémité ou zone et/ou ladite première face et/ou dans lequel ledit élément de déclenchement (124, 224, 324, 424, 224a') est positionné au niveau de ladite deuxième face dudit support (123a1, 223a1, 323a1, 423a1, 223a1') ou au niveau de ladite poignée (112, 212, 312, 412).

19. Moule selon l'une quelconque des revendications précédentes, comprenant des moyens de charnière (130, 230, 330, 430) positionnés dans ladite première demi-coque (102, 202, 302, 402) et/ou dans ladite deuxième demi-coque (104, 204, 304, 404), par exemple positionnés au niveau de la zone arrière pendant l'utilisation (102b2, 202b2, 302b2, 402b2) de ladite première demi-coque (102, 202, 302, 402) et/ou dans ladite zone arrière pendant l'utilisation (104b2, 204b2, 304b2, 404b2) de ladite deuxième demi-coque (104, 204, 304, 404), dans lequel lesdits moyens de charnière (130, 230, 330, 430) permettent la rotation réciproque de ladite deuxième demi-coque (104, 204, 304, 404) par rapport à ladite première demi-coque (102, 202, 302, 402).

20. Moule selon l'une quelconque des revendications précédentes, comprenant des moyens d'extraction (132, 232, 332, 432, 232') du type unidirectionnel ou sans retour, adaptés pour extraire le dispositif d'espacement après son formage et pour rendre ledit moule (100, 200, 300, 400) jetable, dans lequel lesdits moyens d'extraction (132, 232, 332, 432) comprennent un élément de bouton (132a, 232a, 332a, 432a) doté d'une base (132b, 232b, 332b, 432b), apte à être actionné pendant l'utilisation pour l'extraction du dispositif d'espacement formé, et un bout ou extrémité (132c, 232c, 332c, 432c) d'un corps de pression (132c1, 232c1, 332c1, 432c1), ce dernier ayant une conformation sensiblement d'aiguille ou de cheville, dans lequel ledit bout ou extrémité (132c, 232c, 332c, 432c, 232c') est orienté vers ladite cavité (106, 206, 306, 406) et/ou vers ladite cavité supplémentaire (106a) ou dans lequel lesdits moyens d'extraction (232') comprennent un corps de pression (32c1) comprenant une base (32b) et un bout ou extrémité (32c), dans lequel ledit corps de pression (32cl) présente un filetage, adapté pour s'engager avec un siège (33) spécial doté d'une vis de blocage et situé au niveau de ladite première demi-coque (2), comprenant en outre un capuchon ou couvercle (34) pouvant être logé ou positionné au niveau du bout (32c) et dans un siège ou ouverture (34a) approprié situé au niveau de ladite première surface de formage (108, 208, 308, 408), et/ou un élément de poignée (35) pouvant être saisi par un utilisateur dans le but de visser les moyens d'extraction (232') ou comprenant une base en forme de disque (36) adaptée pour comprendre au moins une paire de dents (36a) constituées d'un matériau flexible, adaptées pour permettre la rotation des moyens d'extraction (232'), mais sans permettre leur dévissage.

21. Moule selon l'une quelconque des revendications précédentes lorsqu'elle dépend de la revendication 2, dans lequel ledit canal (150) présente un développement correspondant à celui dudit premier bord périmétrique (107, 207, 307, 407) et/ou dudit deuxième bord périmétrique (109, 209, 309, 409) et de ladite première (108, 208, 308, 408) et/ou de ladite deuxième surface de formage (110, 210, 310, 410), dans lequel ledit canal (150) présente une surface, au moins au niveau du fond, moletée ou présentant une certaine rugosité ou texture ou finition, et dans lequel ledit canal (150) présente une cavité renfoncée par rapport à la surface de ladite première demi-coque ou corps inférieur (102, 202, 302, 402) et/ou de ladite deuxième demi-coque ou corps supérieur (104, 204, 304). 404), avec une paroi sensiblement perpendiculaire ou inclinée par rapport à cette dernière.

22. Procédé de formage d'un dispositif d'espacement pour le remplacement d'une prothèse articulaire, tel qu'un dispositif d'espacement tibial du genou, adapté pour être implanté pendant l'utilisation au niveau de l'os tibial de l'articulation du genou, un dispositif d'espacement fémoral du genou, adapté pour être implanté pendant l'utilisation au niveau de l'os fémoral de l'articulation du genou, un dispositif d'espacement de la hanche, adapté pour être implanté pendant l'utilisation au niveau de l'os fémoral de l'articulation de la hanche, ou un dispositif d'espacement de l'épaule, adapté pour être implanté pendant l'utilisation au niveau d'une articulation de l'épaule, comprenant les phases suivantes :
fournir un moule (100, 200, 300, 400) selon l'une quelconque des revendications 1 à 21, comprenant une première demi-coque ou premier corps inférieur (102, 202, 302, 402) comprenant une première surface de formage (108, 208, 308, 408) délimitée par un premier bord périmétrique (107, 207, 307, 407) et destinée à mouler au moins une première portion dudit dispositif d'espacement, ledit premier bord périmétrique (107, 207, 307, 407) étant continu sur toute ladite première surface de formage (108, 208, 308, 408), et
une deuxième demi-coque ou corps supérieur (104, 204, 304, 404) comprenant une deuxième surface de formage (110, 210, 310, 410) délimitée par un deuxième bord périmétrique (109, 209, 309, 409) et destinée à mouler au moins une deuxième portion dudit dispositif d'espacement, ledit deuxième bord périmétrique (109, 209, 309, 409) étant continu sur toute ladite deuxième surface de formage (110, 210, 310, 410),
fournir un matériau qui constitue ledit dispositif d'espacement et positionner et/ou verser, éventuellement de manière manuelle, ledit matériau dans ladite première surface de formage (108, 208, 308, 408) et/ou dans ladite deuxième surface de formage (110, 210, 310, 410),
coupler ladite première demi-coque ou corps inférieur (102, 202, 302, 402) et ladite deuxième demi-coque ou corps supérieur (104, 204, 304, 404),
amener ledit premier bord périmétrique (107, 207, 307, 407) en butée contre ledit deuxième bord périmétrique (109, 209, 309, 409), afin de fermer ledit moule (100, 200, 300, 400) et délimiter entre ces derniers, au niveau de ladite première surface de formage (108, 208, 308, 408) et de ladite deuxième surface de formage (110, 210, 310, 410), une cavité (106, 206, 306, 406) correspondant à la configuration extérieure du dispositif d'espacement,
attendre un temps de polymérisation et/ou de durcissement dudit matériau et de formage dudit dispositif d'espacement,
dans lequel pendant ladite étape de formage au moins l'un dudit premier bord périmétrique (107, 207, 307, 407) et dudit deuxième bord périmétrique (109, 209, 309, 409) coupe et/ou élimine les bavures de moulage causées par la formation dudit dispositif d'espacement.

23. Procédé selon la revendication 22, dans lequel, pendant ladite étape de formage dudit dispositif d'espacement, une partie du matériau dudit dispositif d'espacement s'écoule ou goutte dans un canal (150) adjacent à au moins l'un dudit premier bord périmétrique (107, 207, 307, 407) et dudit deuxième bord périmétrique (109, 209, 309, 409) et à au moins l'une de ladite première surface de formage (108, 208, 308, 408) et ladite deuxième surface de formage (110, 210, 310, 410) et adhère à et dans ledit canal (150), afin d'éliminer toute bavure de moulage causée par la formation dudit dispositif d'espacement.

24. Procédé selon la revendication 22, dans lequel ladite étape de couplage comprend actionner une poignée (112, 212, 312, 412), par exemple manuellement, et/ou actionner une structure de contrainte amovible (122, 222, 322, 422, 222'), existant dans ladite deuxième demi-coque (104, 204, 304, 404) et/ou dans ladite première demi-coque (102, 202, 302, 402), et contraindre de manière amovible ladite première demi-coque (102, 202, 302, 402) avec ladite deuxième demi-coque (104, 204, 304, 404).

25. Procédé selon la revendication 24, dans lequel ladite étape d'actionnement comprend activer des moyens de couplage (123, 223, 323, 423, 223'), par exemple à encliquetage, du type amovible de ladite structure de contrainte amovible (122, 222, 322, 422, 222') et/ou dans lequel ladite étape d'actionnement des moyens de couplage (123, 223, 323, 423, 223') comprend porter un élément de dent ou de protubérance (123a, 223a, 323a, 423a, 223a'), existant au niveau de ladite deuxième demi-coque (104, 204, 304, 404) au niveau d'un siège d'engagement approprié (123b, 223b, 323b, 423b, 223b'), présentant une conformation correspondant ou complémentaire à celle dudit élément de dent ou de protubérance (123a, 223a, 323a, 423a, 223a'), réalisé dans ladite première demi-coque (102, 202, 302, 402) ou vice versa et engageant, par exemple par encliquetage, ledit élément de dent ou de protubérance (123a, 223a, 323a, 423a, 223a') dans ledit siège d'engagement (123b, 223b, 323b, 423b, 223b').

26. Procédé selon l'une quelconque des revendications 22 à 25, comprenant une étape d'extraction du dispositif d'espacement formé par ledit moule (100, 200, 300, 400), dans lequel ladite étape d'extraction comprend éloigner l'une de l'autre ladite première demi-coque (102, 202, 302, 402) et ladite deuxième demi-coque (104, 204, 304, 404), libérer lesdits moyens de couplage (123, 223, 323, 423, 223') faisant sortir ledit élément de dent ou de protubérance (123a, 223a, 323a, 423a, 223a') dudit siège d'engagement (123b, 223b, 323b, 423b, 223b'), et/ou agir sur un élément de déclenchement (124, 224, 324, 424, 224a') de ladite structure de contrainte amovible (122, 222, 322, 422, 222') et/ou sur ladite poignée (112, 212, 312, 412) pour ladite étape d'éloignement et de libération.

27. Procédé selon la revendication précédente, dans lequel ladite étape d'extraction comprend actionner les moyens d'extraction de type unidirectionnel ou anti-retour (132, 232, 332, 432, 232'), aptes à extraire le dispositif d'espacement après son formage et à rendre ledit moule jetable (100, 200, 300, 400), dans lequel ladite étape d'actionnement comprend presser une base (132b, 232b, 332b, 432b) d'un élément de bouton (132a, 232a, 332a, 432a) de sorte qu'un bout ou extrémité (132c, 232c, 332c, 432c) d'un corps de pression (132c1, 232c1, 332c1, 432c1) dudit élément de bouton (132a, 232a, 332a, 432a), dans lequel ledit bout ou extrémité (132c, 232c, 332c, 432c) est orienté vers ladite cavité (106, 206, 306, 406) et/ou dans ladite cavité supplémentaire (106a), pousse ledit dispositif d'espacement hors de ladite cavité (106, 206, 306, 406) et/ou de ladite cavité supplémentaire (106a) ou dans lequel ladite étape d'actionnement comprend visser un corps de pression (32c1) dans un siège spécial (33) réalisé dans ladite première demi-coque (102, 202, 302, 402), de sorte qu'un bout ou extrémité (32c) dudit corps de pression (32c1) orienté vers ladite cavité (106, 206, 306, 406) et/ou vers ladite cavité supplémentaire (106a), pousse ledit dispositif d'espacement hors de ladite cavité (106, 206, 306, 406) et/ou de ladite cavité supplémentaire (106a).
